(19) **Europäisches Patentamt**
**European Patent Office**
**Office européen des brevets**

(11) **EP 4 725 303 A1**

(12) **EUROPEAN PATENT APPLICATION**

(43) Date of publication:
15.04.2026  Bulletin 2026/16

(21) Application number: 24206058.0

(22) Date of filing: 11.10.2024

(51) International Patent Classification (IPC):
*A01H 5/10* (2018.01)     *A01H 6/46* (2018.01)

(52) Cooperative Patent Classification (CPC):
**A01H 6/4672; A01H 5/10;** C12Q 1/6895;
C12Q 2600/13

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Agrokray Limited Liability Company
31000 Krasyliv Khmelnytsky (UA)**

(72) Inventors:
- **Petrovych, Biliavets Oleh
  31000 Krasyliv City, Khmelnytsky Region (UA)**
- **Illich, Rybalka Oleksandr
  65036 Odesa City (UA)**
- **Yakovych, Kosiuk Vasyl
  31047 Krasyliv district, Khmelnytsky Region
  (UA)**

(74) Representative: **Bishop, Ian Keith
  Asesores Juridicos Valeria IP, S.L.
  Av. Diagonal 640 / 6 Planta A
  08017 Barcelona (ES)**

(54) **SPELT, METHOD OF PRODUCING SPELT PLANTS, GRAIN PRODUCED FROM SPELT PLANT AND FOOD PRODUCT PRODUCED THEREFROM**

(57)     The subject invention covers a seed of Triticum spelta (Bilberry) variety, representative seed of said variety has been deposited under Number NCIMB 44437 , plant produced thereof, methods of breeding, commodity plant produced therefrom as well as metho d of producing commodity plant product, grain produced therefrom and food product produced from grain. The main feature of the invention is that initial grain colour with violet subtones and higher anthocyanin content are intrinsic to seeds and is conferred from grain to the final food product.

Description

FIELD OF INVENTION

This invention is in the field of spelt (*Triticum spelta L.*) breeding, specifically relating to a spelt variety designated BILBERRY.

BACKGROUND OF INVENTION

There are numerous steps involving significant intervention in the development of any novel, desirable plant germplasm. The goal is to combine in a single variety an improved combination of desirable traits from the parental germplasm. These traits may include, but are not limited to, higher seed yield, resistance to diseases and/or insects, tolerance to drought and/or heat, altered milling properties, abiotic stress tolerance, improvements in compositional traits, and better agronomic characteristics.

One of the most grown worldwide and is the most widely adapted cereal is wheat. There are five main wheat market classes. They include the four common wheat (*Triticum aestivum* L.) classes: hard red winter, hard red spring, soft red winter, and white (hard and soft). The fifth class is durum (*Triticum turgidum L.*). Common wheats are used in a variety of food products such as bread, cookies, cakes, crackers, and noodles. In general the hard wheat classes are milled into flour used for breads and the soft wheat classes are milled into flour used for pastries and crackers.

Many of the breads and other pastry products are not well tolerated by people with illnesses such as impaired stomach and intestinal function or candidiasis, because after eating the bread, symptoms such as flatulence, constipation, diarrhea, bloating, heartburn, etc. often occur. Studies suggest that between 0.2% and 1.3% of the world population has a wheat allergy. If someone has a wheat allergy, a body responds by creating immunoglobulin E (IgE) after consuming wheat. Human body makes many different types of IgE, which are sensitive to specific kinds of allergens. Also non-IgE reactions involve immune system. Eeactions may include eosinophilic esophagitis (EoE) or eosinophilic gastritis (EG). EoE causes inflammation in esophagus, EG causes inflammation in stomach lining. There are four types of protein that normally are allergic in the wheat, such as albumin, gliadin, globulin, gluten. Moreover, in case of bread and pastry products, allergies may be even more significant due to the presence of yeast.

It is unknown exactly when and by whom yeast was first added to bread to make it rise. It is not precluded that this can be traced back all the way to the Nile Valley during the time of the pharaohs. What is certain is that the later Egyptians had leavened bread, and bread with and without sourdough is referred to in the Old Testament, in the Exodus from Egypt.

Wheat naturally contains various types of wild yeast cell. The density of these yeast cells is however so low that dough would not rise, or hardly so, just from these cells. It is however possible to cause an increase of the concentration of yeast cells in a natural manner. The only requirement needed to start a multiplication process is to add water to flour and to leave it while sufficient oxygen has access thereto. Due to the acetic acid bacteria and lactic acid bacteria naturally present in flour, the acidity of the mixture increases, which is why this mixture is usually referred to as sourdough, or leaven or culture. After a number of days, a sourdough can thus be grown with a concentration of yeast cells which is high enough to thereby cause a dough to rise so as to be able to bake a loaf of bread therefrom. For this purpose this acidic dough is mixed with more flour, and the mixture must then rest for at least one more day. A portion of the acidic dough can be used as starter for a subsequent quantity of leaven. This is generally done in order to keep ending up with the same or at least a similar sourdough culture, and thereby obtain a consistent flavour of the final product.

When yeast is added to the dough mixture, the yeast cells will multiply therein. This increase in the number of yeast cells is accompanied by a conversion of sugars in the dough, wherein carbon dioxide is formed by the yeast cells. This process is also referred to as digestion or fermentation. The formed gas bubbles are trapped in the dough, with the result that the dough takes on a light, airy structure as is familiar and appreciated in a well-risen loaf of bread.

In sourdough, the density of yeast cells will however never equal the level of baker's yeast cultivated specifically for this purpose. This is a so-called pure culture, often of one type of yeast, usually Saccharomyces Cerevisiae, which is most commonly applied in bread owing to its high carbon dioxide-producing capacity. One kilogram thereof contains in the order of ten billion yeast cells, while a kilogram of flour often naturally comprises no more than several tens of thousands of yeast cells. Sourdough bread is therefore generally less airy in that the dough has been able to rise less. In certain embodiments, the present invention has for its object, among others, to prepare a dough and to provide a dough and a bread product baked therefrom on the basis of leaven which provides an increased rising capacity.

Wheat and wheat-grass are reported to possess antimicrobial activity against many common human pathogens [10,11]. Choi et al. [12] found wheat seed ethyl acetate extracts of different wheat varieties to be effective against the pathogens *Escherichia coli, Salmonella typhimurium,* and *Staphylococcus aureus* using well diffusion assays. Kim et al. [13] studied the antimicrobial effect of wheat germ against *S aureus, E. coli, S. typhimurium,* and *Bacillus cereus* and found the potential of wheat germ as a natural antimicrobial and food preservative agent. Wheat-grass extracts are also reported to show antibacterial activity against many bacteria like *Yersinia enterocolitica* and *Listeria monocytogenes,* which are some foodborne pathogens [14,15]. Ashok [16] also reported the antibacterial activity of wheat-grass juice against the pathogens E. *coli, Pseudomonas aeruginosa, S aureus,* and *Candida albicans.*

Therefore, there is a need in new plant variety that can be alternative to wheat varieties, but having low allergy impact while saving consumer attractive characteristics. This includes a need in seeds of appropriate plant variety, commodity plant produced therefrom, grain produced from the commodity plant and food product produced from it.

**PRIOR ART**

Prior art includes CN100334944C that provides method for improving the breeding efficiency of hybrid wheat for killing hybrid wheat, characterized in that the method includes the following steps:

1), Breeding of basic tool materials for spelt;

① Taking Spelt [S (mm)] as the female parent, cross with multi-ovary wheat [A (MM)] to obtain single-ovary plant F1 [S (Mm)], where S represents Spelt wheat Cytoplasm, m represents the single ovary gene in the nucleus of Spelt wheat, A represents the cytoplasm of common wheat, and M represents the ovary gene in the nucleus of common wheat;

②Single ovary plants F1 [S (Mm)] selfed and obtained F2, the ovary traits were separated, the plants showing single ovary [S (Mm)] and [S (mm)] were eliminated, and the plants showing multi ovary [S (MM)] Reserved, which is the basic tool material of the multi-ovary Spelt wheat, continue to be self-purified for use as a variety of other common wheat to be transformed into varieties with multiple ovary traits or with multiple ovary traits The female parent of the strain;

2), Multi-ovary wheat varieties or strains with Spelt cytoplasm

①Taking the basic tool material of multi-child house Spelt [S (MM)] as the female parent, the existing female parent Xinong 1376 [A (mm)] as the parent parent Crossing to obtain ovary plant F1 [S (Mm)];

②Single ovary plant F1 [S (Mm)] is the female parent, Xinong 1376 [A (mm)] is the reincarnation parent for backcrossing, and the single ovary plant BC1 [S (Mm) + S (mm)] is obtained;

③Single ovary plant BC1 [S (Mm) + S (mm)] is self-breeding, and BC1 F1 [S (mm) + S (Mm) + S (MM)] is obtained. The ovary traits are separated. Ovary trait plant [S (MM)] as the parent to continue backcrossing;

④ From the BC1 F1 plants, select plants with similar traits to the reincarnation parent Xinong 1376, and exhibit multi-ovary traits [S (MM)] backcross with the reincarnation parents to obtain BC2 [S (Mm)], representing single ovary;

⑤ Continue to select plants similar to the traits of the reincarnation parent Xinong 1376 in BC2 [S (Mm)] and backcross with the reincarnation parent to obtain BC3 [S (Mm) + S (mm)], the ovary trait genotype is separated , Self-breeding of the fixed plant, from which the plant with multiple ovary traits [S (MM)] is still selected as the parent to continue backcrossing;

⑥ In step ③ ~ step ⑤, pay attention to selecting plants with similar traits of reincarnation parents and showing multi-ovary traits [S (MM)] to continue backcrossing with reincarnation parents until BC4 - BC6, the gene is obtained Descendants of the type [S (Mm) + S (mm)], and the external appearance is a single room;

⑦ Select plants with genotypes [S (Mm) + S (mm)] from BC4 to BC6 that are similar to reincarnate parental traits and exhibit multi-ovary traits [S (MM)], that is, have Spears The excellent wheat cultivars with multiple ovary wheat or the excellent wheat cultivars with Spelt cytoplasm, except that the wheat exhibits multiple ovary traits, the other traits are the same as the original father Xinong 1376;

3), Multi-Ovary Wheat, Female Breeding, Breeding of Strong Dominant Hybrid Combination and Hybrid Seed Production

① In the multi-ovary wheat female parent breeding area, the excellent multi-ovary wheat [S (MM)] with Spelt wheat cytoplasm through continuous self-breeding from generation to generation, continue to obtain more Spelt wheat cytoplasm Superior ovary wheat varieties or superior ovary wheat lines with Spelt wheat cytoplasm [S (MM)];

②In the hybrid seed production area of multi-ovary wheat, the excellent multi-ovary wheat [S (MM)] with the cytoplasm of Spelt wheat is used as the female parent, and the parent [A (mm)] group is combined with the predominant killing strength Combined with hybrid seed production to improve the reproduction coefficient of the original seed, the hybrids obtained by seed production [S (Mm)] all appear as single ovary in production.

The main drawback is the complexity of obtaining plant varieties without accessing another benefits in the obtained variety, such as consumer attractiveness.

DE1 9809547A1 provides using of spelt commodities for obtaining a spelt crispbread made from a dough which

(i) 100 parts by weight of spelt flour and, based on the quantity of flour,

(ii) 65 to 90 parts by weight of liquid,

(iii) 5 to 10 parts by weight of oil, clarified butter, butter, clarified butter and/or salted butter and, where appropriate,

(iv) 10 to 20 parts by weight of sesame,

(v) up to 1 part by weight of natural additives.

In certain embodiments, this invention provides benefits to the persons suffering from allergy to wheat and yeast, but it provides the texture, flavour and taste of crispbread rather when conventional bread, so, the sphere of use is limited.

RS20150805A1 provides a protein wholegrain bread from spelt with the inclusion of a plant protein derivative, which is the pea protein isolate. Pea proteins are a popular choice for vegetarians and vegans alike. Pea proteins are anti-allergic, they have a 98% digestion rate, which means the body is able to absorb the vast majority of foods that contain pea proteins. The success of the subject procedure, which includes natural raw material (pea protein) in the recipe, which contributes to the improvement of the rheology of the integral spelled flour dough, the production of high-protein integral spelled bread with pronounced freshness, and unexpectedly contributes to adequate sensory properties of bread.

However, inclusion of pea protein as a raw materials makes technology more difficult.

## SUMMARY OF THE INVENTION

In a first broad independent aspect, an embodiment of the invention provides a food product comprising a seed, a plant part or a flour obtained from a seed of a Triticum spelta (Bilberry) variety, wherein representative seed of said variety has been deposited under Number NCIMB 44437. This configuration is particularly advantageous for its anti-allergenic properties and/or anti-cancer properties.

In a subsidiary aspect in accordance with the preceding broad aspect, the Tritcum spelta (Bilberry) variety has an anthocyanin content of 60-80 mg/kg; whereby it acts as an antiallergenic.

In a further broad aspect, an embodiment of the invention provides a converted seed, plant, plant part or plant cell product obtained from a Triticum spelta (Bilberry) variety, wherein representative seed of the variety (Bilberry) having been deposited under number NCIMB 44437, wherein the converted seed, plant, plant part or plant cell comprises a locus conversion, and wherein the plant or a plant grown from the converted seed, plant part or plant cell comprises the locus conversion and otherwise comprises essentially all of the physiological and morphological characteristics of the seed, plant, plant part or plant cell of variety (Bilberry) when grown under the same environmental conditions.

In a further subsidiary aspect, an embodiment of the invention provides a method of producing a genetic marker profile comprising extracting nucleic acids from the seed of any of the preceding aspects or the plant germinated from said seed and genotyping said nucleic acids at one or more genetic loci, thereby producing a genetic marker profile.

In a further subsidiary aspect, the method of plant breeding comprises a) isolating nucleic acids from the seed of any of the preceding aspects or a plant germinated from said seed, b) identifying one or more polymorphisms from the isolated nucleic acids, and c) selecting said seed or a plant obtained from said seed having said one or more polymorphisms, wherein the seed or plant is used in a plant breeding method.

In a further broad aspect, an embodiment of the invention provides a method of producing bread by selecting flour derived from a seed of a Triticum spelta (Bilberry) variety, wherein representative seed of said variety has been deposited under Number NCIMB 44437.

In a further broad aspect, an embodiment of the invention provides a seed of Triticum spelta (Bilberry) variety, wherein representative seed of said variety has been deposited under Number NCIMB 44437.

In a further broad aspect, an embodiment of the invention provides a plant or part of the plant of variety (Bilberry), wherein representative seed of said variety (Bilberry) has been deposited under Number NCIMB 44437.

In a further broad aspect, an embodiment of the invention provides a seed produced on the plant of any of the preceding aspects.

In a further subsidiary aspect, an embodiment of the invention provides a process for producing seed, said process comprising crossing the plant of any of the preceding aspects with itself, and harvesting the seed.

In a further subsidiary aspect, an embodiment of the invention provides an F1 seed produced by the process of any of the preceding aspects or an F1 plant produced by germinating the seed of any of the preceding aspects.

In a further aspect, an embodiment of the invention provides a method of producing a genetic marker profile comprising extracting nucleic acids from the seed of any of the preceding aspect or the plant germinated from said seed and genotyping said nucleic acids at one or more genetic loci, thereby producing a genetic marker profile.

In a further subsidiary aspect, an embodiment of the invention provides a method of plant breeding comprising a) isolating nucleic acids from the seed of any of the preceding aspects or a plant germinated from said seed, b) identifying one or more polymorphisms from the isolated nucleic acids, and c) selecting said seed or a plant obtained from said seed having said one or more polymorphisms, wherein the seed or plant is used in a plant breeding method.

In a further subsidiary aspect, an embodiment of the invention provides a plant produced by the process of any of the preceding aspects.

In a further aspect, an embodiment of the invention provides a method of producing a plant derived from variety NCIMB 44437, the method comprising the steps of (a) growing the plant of any of the preceding aspects; (b) crossing said plant with itself to produce a seed of a progeny plant; (c) repeating step (b) at least one or more times; and (d) growing said progeny plant from said seed and crossing the progeny plant with itself to produce a plant derived from variety NCIMB 44437.

In a further broad aspect, an embodiment of the invention provides a seed of Triticum spelta (Bilberry) variety, wherein representative seed of said variety has been deposited under Number NCIMB 44437.

In a further broad aspect, an embodiment of the invention provides a plant of variety (Bilberry), wherein representative seed of said variety (Bilberry) has been deposited under Number NCIMB 44437.

In a further broad aspect, an embodiment of the invention provides a plant part of the plant in accordance with any preceding aspect.

In a further aspect, a seed may be produced on the plant of any of the preceding aspects.

In a further broad aspect, a converted seed, plant, plant part or plant cell of variety (Bilberry), representative seed of the variety (Bilberry) having been deposited under number NCIMB 44437, wherein the converted seed, plant, plant part or plant cell comprises a locus conversion, and wherein the plant or a plant grown from the converted seed, plant part or plant cell comprises the locus conversion and otherwise comprises essentially all of the physiological and morphological characteristics of the seed, plant, plant part or plant cell of variety (Bilberry) when grown under the same environmental conditions.

In a further subsidiary aspect, an embodiment of the invention provides a process for producing seed, said process comprising crossing the plant of any preceding aspect with itself, and harvesting the seed.

In a further subsidiary aspect, an F1 seed produced by the process of any of the preceding aspects.

In a further subsidiary aspect, an F1 plant produced by germinating the seed of any preceding aspect.

In a further subsidiary aspect, a method of producing a genetic marker profile comprises extracting nucleic acids from the seed of any preceding aspect or the plant germinated from said seed and genotyping said nucleic acids at one or more genetic loci, thereby producing a genetic marker profile.

In a further broad aspect, a method of plant breeding comprises a) isolating nucleic acids from the seed of claim 1 or a plant germinated from said seed, b) identifying one or more polymorphisms from the isolated nucleic acids, and c) selecting said seed or a plant obtained from said seed having said one or more polymorphisms, wherein the seed or plant is used in a plant breeding method.

In a further subsidiary aspect, an embodiment of the invention provides a plant produced by the process of any of the preceding aspects.

In a further broad aspect, a method of producing a plant derived from variety NCIMB 44437, the method comprising the steps of (a) growing the plant of any of the preceding aspects; (b) crossing said plant with itself to produce a seed of a progeny plant; (c) repeating step (b) at least one or more times; and (d) growing said progeny plant from said seed and crossing the progeny plant with itself to produce a plant derived from variety NCIMB 44437.

In a further subsidiary aspect, the method of producing a commodity plant product comprises collecting the commodity plant product from the plant of any preceding aspect.

In a further broad aspect, an embodiment of the invention provides grain produced from a commodity plant product from the plant of any preceding claim wherein anthocyanin content is 60-80 mg/kg providing brown colour with violet subtones.

In a further subsidiary aspect, an embodiment of the invention provides the food product produced from a grain of any preceding aspect with initial brown grain colour with violet subtones and higher anthocyanin content conferred from grain.

In a further subsidiary aspect, the method of producing food product comprises the step of preparing dough with use of wholegrain flour produced from brown grain with violet subtones and higher anthocyanin content conferring anti-allergenic or low allergic effect.

The term Bilberry may also be referred to as BILB. Also provided are plants produced by growing the seed of the spelt variety Bilberry, as well as the derivatives of such plants. Further provided are plant parts, including cells, plant protoplasts, plant cells of a tissue culture from which spelt plants can be regenerated, plant calli, plant clumps, and plant cells that are intact in plants or parts of plants, such as leaves, stems, roots, root tips, anthers, pistils, seed, grain, pericarp, embryo, pollen, ovules, cotyledon, hypocotyl, spike, floret, awn, lemma, shoot, tissue, petiole, cells, and meristematic cells, and the like. In certain embodiments, the invention provides an alternative to wheat varieties whilst minimizing or even entirely avoiding the risk of allergic reaction to products containing or derived from the claimed subject matter.

In a second aspect, a tissue culture of regenerable cells of the spelt variety Bilberry is provided, as well as plants and plant parts regenerated therefrom, wherein the regenerated spelt plant is capable of expressing all the physiological and morphological characteristics of a plant grown from the spelt seed designated BILBERRY. A spelt plant comprising a locus conversion or single locus conversion of the spelt variety Bilberry, wherein the spelt plant is otherwise capable of expressing all the physiological and morphological, or phenotypic, characteristics of the spelt variety Bilberry is provided. The locus conversion may comprise, for example, a transgenic gene which has been introduced by genetic transformation into the spelt variety Bilberry or a progenitor thereof. The locus conversion may, for example, comprise a dominant or recessive allele or a genetic modification introduced by manipulation of the plant genome. The locus conversion may confer potentially any trait upon the converted plant, including, but not limited to, herbicide resistance, insect resistance, resistance to bacterial, fungal, or viral disease, male fertility or sterility, abiotic stress, altered phosphorus content, altered antioxidants, altered essential amino acids, and altered nutritional quality, such as altered starch, sugars, non- digestible carbohydrate, protein, oil or fatty acids. The altered trait can be compared to a spelt variety BILBERRY not comprising the locus conversion.

Spelt plants are provided which comprise a transgene or genetic modification and which were produced by transforming or modifying the plant, plant part, seed or cell of spelt variety BILBERRY, or which had the transgene or the genetic modification introgressed through back-crossing.

Methods for producing a spelt plant are provided in which plant breeding techniques are applied to a spelt plant grown from seed of the pelt variety BILBERRY comprising a locus conversion, or to a plant grown from seed of a cross of such a spelt plant to a different plant, namely common wheat. First generation (F1) hybrid wheat seed produced by crossing a plant of the spelt variety Bilberry to a second plant, namely common wheat plant, are provided. Also provided are the F1 hybrid spelt plants grown from the hybrid seed produced by crossing the spelt variety Bilberry to a second plant, namely common wheat plant. For example, the method of crossing can comprise the steps of: (a) planting seeds of the spelt variety Bilberry; (b) cultivating spelt plants resulting from said seeds until said plants bear flowers; (c) allowing fertilization of the flowers of said plants; and (d) harvesting seeds produced from said plants. In the method, the plant breeding techniques may be selected, for example, from recurrent selection, mass selection, bulk selection, backcrossing, pedigree breeding, genetic marker- assisted selection and genetic transformation. The spelt plant of variety BILBERRY may be used as the male or female parent.

A method of producing a spelt plant derived from the spelt variety Bilberry is provided, the method comprising the steps of: (a) preparing a progeny plant derived from the spelt variety BILBERRY by crossing a plant of the spelt variety Bilberry with a second spelt plant; and (b) crossing the progeny plant with itself or a second plant to produce a progeny plant of a subsequent generation which is derived from a plant of the spelt variety BILBERRY. Optionally, the method may further comprise: (c) crossing the progeny plant of a subsequent generation with itself or a second plant; and (d) repeating steps (b) and (c) for at least, for example 2, 3, 4 or more additional generations to produce an inbred spelt plant derived from the spelt variety Bilberry. Also provided is a plant produced by this and other methods described herein. A method of producing a spelt plant derived from the spelt variety Bilberry can, for example, further comprise: (a) crossing the spelt variety BILBERRY- derived spelt plant with itself or another spelt plant to yield additional spelt variety BILBERRY-derived progeny spelt seed; (b) growing the progeny spelt seed of step (a) under plant growth conditions to yield additional spelt variety BILBERRY- derived spelt plants; and (c) repeating the crossing and growing steps of (a) and (b) to generate further spelt variety BILBERRY-derived spelt plants., Steps (a) and (b) can be repeated if desired at least 1, 2, 3, 4, or 5 or more times.

Also provided is a spelt plant produced by this and other methods described herein. Methods for producing double haploid spelt plants from spelt variety BILBERRY are provided. For example, a spelt plant produced by growing a seed of the cross of spelt variety BILBERRY with a different spelt plant or plant part can be crossed with another plant to form haploid cells. The chromosomes of the haploid cells can be doubled to form double haploid cells which are grown into a double haploid spelt plant or plant part. Haploid seed generated from a cross of a spelt plant disclosed herein with a different spelt plant can be doubled to produce a spelt plant having doubled haploid chromosomes.

In another aspect, an embodiment of the present invention may concern grain produced from a commodity spelt plant product having intrinsic characteristics of the spelt plant belonging to BILBERRY variety.

In a further aspect, an embodiment of the invention concerns food product produced from grain of spelt plant BILBERRY saving the having intrinsic characteristics of the spelt plant belonging to BILBERRY variety.

## DETAILED DESCRIPTION

In at least one embodiment, the present invention relates to a new and distinctive spelt (*Triticum spelta L.*) variety designated BILBERRY, its seeds, plants, plant parts and hybrids. Variety BILBERRY represents a significant advancement in elite germplasm. Also provided are methods for obtaining the spelt variety BILBERRY that comprise crossing common wheat variety Chornobrova (originated in Ukraine) with spelt variety Schwabenkorn (originated in Germany). Processes for making a spelt plant containing in its genetic material one or more traits introgressed into BILBERRY through backcross conversion and/or transformation or genetic modification, and to the wheat seed, plant and plant parts produced thereby.

*Triticum spelta,* a collection sample from Germany, Schwabenkorn, is a typical spelt with a spelt-shaped ear up to 145 cm in plant height, prone to laying down and shedding of the ear. It is the donor of the purple grain trait.

Winter common wheat variety CHORNOBROVA has a dark purple grain. The variety is 110 cm tall, the ear is spiny, the grain is dark brown. Resistant to the main plant diseases.

5 interrupted backcrosses (BC5) were performed on the original spelt as a parent component with periodic selections of plants of the spelta morphotype. A short-stemmed recombinant with slightly better grain milling than the original spelled was selected from the offspring of VS4(F2). In the course of the selection program, a unique short-stemmed spelled with dark brown (black) grain was created. Currently there are no other known analogues in the world practice of creating similar spelled with black grain.

The plant variety provides plant with mass weight 1000 - 45g, protein content - 15.34%, gluten content 36.88%). Other characteristics are shown in the table 1.

Table 1

|   | Feature | Expression | Code |
|---|---------|-----------|------|
| 1 | Coleoptile: intensity of anthocyanin color | High | 7 |
| 2 | Plant (habitus) | Semi-straight | 3 |
| 3 | Flag leaf (anthocyanin color of ears) | Absent or very weak | 1 |
| 4 | Quantity of plants with bended flag leaf | Absent or very low | 1 |
| 5 | Time of ear emergence | Medium | 5 |
| 7 | Ear: glaucosity | weak | 3 |
| 9 | Plant length: (steam, ear) | Medium | 5 |
| 10 | Straw: pit in cross section (halfway between base of ear and stem node below) | Thin | 3 |
| 11 | Ear: shape in profile | Fusiform | 5 |
| 12 | Ear: density | Very low | 1 |
| 13 | Ear: length without awns/scurs | Very long | 9 |
| 14 | Ear: awns or scurs | Scurs | 2 |
| 15 | Ear: length of awns/scurs on the top by length | Very short | 1 |
| 16 | Ear: color | Gray and smoky | 2 |
| 17 | Straw: hairiness of convex surface of upper node | weak | 3 |
| 18 | Lower glume: shoulder width (spikelet) | Wide | 7 |
| 19 | Lower glume: shoulder shape | Straight | 3 |
| 20 | Lower glume: scur length | Very short | 1 |
| 21 | Lower glume: scur shape | Straight | 1 |
| 22 | Lower glume: hairiness on internal surface | Weak | 3 |
| 23 | Lower lemma: scur shape | Moderate bended | 3 |
| 24 | Seed (color) | Other | 5 |

(continued)

|  | Feature | Expression | Code |
|---|---|---|---|
| 25 | Seed: color in phenol | Absent or very light | 1 |
| 31 | Flags leaf: Glaucosity of leaf blade | moderate | 5 |
| 32 | Lower glume: hairiness on external surface | Present | 9 |

It has high frost resistance; Drought resistance is average. Resistance to fungal leaf diseases is average. Other commercial characteristics are shown in the table 2.

Table 2.

| Index | value |
|---|---|
| Yield (standard humidity 14 %, t/ha) | 4,63 - 6,63 |
| Vegetation period, days | 266 - 276 |
| Plant height, sm | 104,1 - 110,8 |
| Weight of 1000 grains, grams | 44,7 - 59,9 |
| Protein content, % | 14 - 15,1 |
| Raw gluten content, % | 27,7 - 31,6 |
| Alveograph score (W) | 84 - 157 |
| Volume of bread from 100 g of flour, m | 740 - 760 |
| Resistance to lodging, code | 5-7 |
| Resistance to shedding, code | 9 |
| Resistance to draught, code | 7 - 8 |
| Resistance to *Erisiphe graminis DC. F.tritici* Marchal., code | 5- 9 |
| Resistance to *Puccinia triticina Erikks.,* code | 7-9 |
| Resistance to *Fusarium graminearum* Schw., code | 9 |
| Resistance to *Oscinella* code | 9 |
| Resistance to *Eurygaster integriceps Put.* code | 8 - 9 |
| Winter hardiness in field, code | 7 - 9 |
| Frost hardiness in artificial frosting | 5.5 |
| Direction of use | cereal |

The provided data shows that the spelt variety Bilberry is unique in its morphological and botanical characteristics and differs from any winter wheat variety as well as from any other spelt variety. The variety differs from the original spelt variety in the height of the plants and the color of the grain.

Grain of Bilberry plant variety has a high content of anthocyanin pigments of purple color (60-80 mg/kg), and due to this, high antioxidant activity.

Anthocyanin is considered as one of the flavonoids, although it has a positive charge at the oxygen atom of the C-ring of basic flavonoid structure. It is also called the flavylium (2-phenylchromenylium) ion. Anthocyanins are present in glycosylated forms of six anthocyanidins, namely, cyanidin, delphinidin, pelargonidin, peonidin, petunidin, and malvidin.

Antocyanins are bioactive compounds with numerous health benefits, including preventing and fighting various chronic diseases such as cancer, cardiovascular diseases, diabetes, inflammation, obesity, aging, liver dysfunction, and hypertension. Besides this, anthocyanins are also reported to possess a strong antimicrobial activity against different Gram-positive and Gram-negative human pathogens . Lacombe et al. demonstrated that anthocyanin-rich American cranberry showed a reduction in the growth of *E. coli* after treatment with anthocyanin extracts. Red cabbage, sour cherry pomace, and *Lonicera caerulea L.* (haskap) berries are good sources of anthocyanins and their extracts are used as natural antimicrobial agents to prevent foodborne outbreaks related to E. coli, S aureus.

Following consumption, anthocyanin, absorption occurs along the gastrointestinal tract, the distal lower bowel being the place where most of the absorption and metabolism occurs. In the intestine, anthocyanins first undergo extensive microbial catabolism followed by absorption and human phase II metabolism. This produces hybrid microbial-human metabolites which are absorbed and subsequently increase the bioavailability of anthocyanins.

Normally cranberry, bilberry, sour cherry are used as a source of antocyanines. However, bread and other pastry are still most important in everyday diet, especially for developing countries, so, there is a need for finding new anthocyanin sources in the cereals.

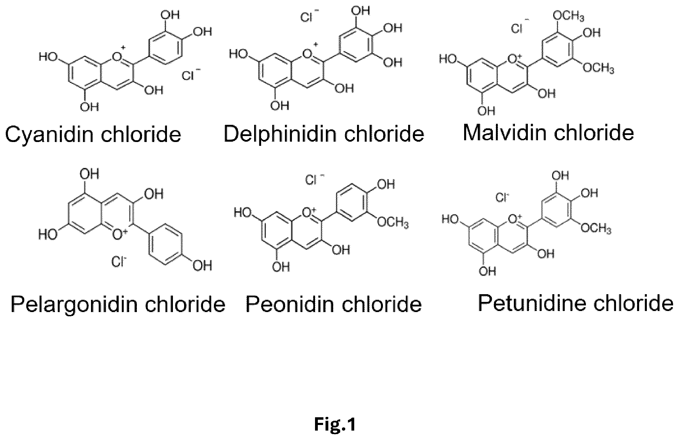

The antocyanins available in the cereals are given in the figure 1.

Commonly cultivated wheat (T. aestivum) is amber in color and has a much fewer amount of anthocyanins, but colored wheat varieties (blue, black, purple, and red) of T. aestivum are rich in anthocyanins and other phytochemicals and are becoming popular around the world nowadays. Colored wheat contains a high amount of anthocyanins.

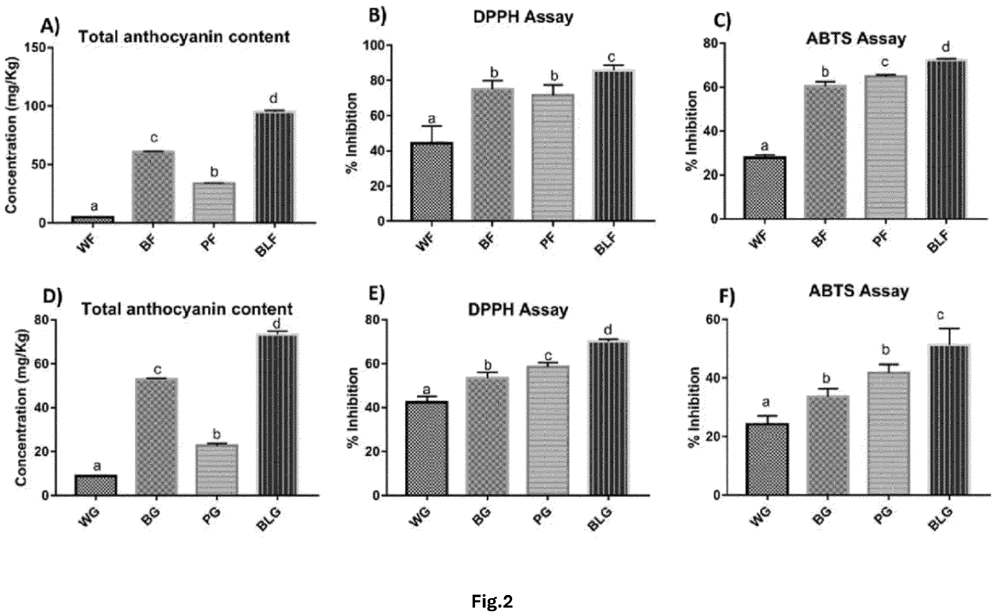

The total anthocyanin content (TAC) of wheat flour ranged between 6.61 and 95.04 mg/kg and followed the order black > blue > purple > white. All colored wheat varieties had significantly higher TAC content than white wheat (Figure 2A). In the case of wheat-grass juice, TAC content ranged between 8.88 and 72.26 mg/kg. The highest TAC content was observed in black wheat-grass juice, followed by blue and purple wheat-grass juice and the lowest was in white wheat-grass juice (Figure 2D). Figure 2 demonstrates total anthocyanin content (A,D); and antioxidant potential of wheat flour and wheat-grass juice using DPPH (2,2-diphenyl-1-picrylhydrazyl) (B,E) and ABTS ((2,20 -azino-bis(3-ethylbenzothiazoline-6-sulfonic acid) (C,F) assays. Each bar represents the mean of three replicates $\pm$ standard deviation. Significant differences in the bar heights analyzed according to one-way ANOVA. Different letters above bars in each graph represent significantly different values ($p < 0.05$) designated with a < b < c. Similar letters above the bars represent values being statistically on par. WF-white wheat flour; BF-blue wheat flour; PF-purple wheat flour; BLF-black wheat flour; WG-white wheat-grass; BG-blue wheat-grass; PG-purple wheat-grass; BLG-black wheat-grass.

Concentration of different anthocyanins present in wheat flour and wheat grass are shown in the Table 3.

Table 3.

| Wheat Flour | | | | |
|---|---|---|---|---|
| Anthocyanins | Black | Purple | Blue | White |
| DGI | 29.14 $\pm$ 0.87[g] | 0.40 $\pm$ 0.02[b] | 4.95 $\pm$ 0.04[f] | 0.03 $\pm$ 0.01[a] |
| DG | 25.14 $\pm$ 0.69[f] | 0.08 $\pm$ 0.00[a] | 0.40 $\pm$ 0.01[a] | - |
| DR | 0.66 $\pm$ 0.06[ab] | - | 2.08 $\pm$ 0.09[c] | - |
| CG | 20.50 $\pm$ 1.06[e] | 2.64 $\pm$ 0.04[e] | 4.50 $\pm$ 0.03[e] | 0.07 $\pm$ 0.01[b] |
| PODG | 0.23 $\pm$ 0.07[a] | - | 0.31 $\pm$ 0.10[a] | - |
| PTG | 2.29 $\pm$ 0.21[c] | - | 3.18 $\pm$ 0.30[d] | - |
| PLG | 2.13 $\pm$ 0.05[c] | 0.88 $\pm$ 0.06[d] | 0.39 $\pm$ 0.09[a] | - |
| POG | 1.40 $\pm$ 0.09[bc] | - | - | - |
| POR | 0.97 $\pm$ 0.10[ab] | - | 0.76 $\pm$ 0.26[b] | - |
| MG | 2.18 $\pm$ 0.17[c] | 1.32 $\pm$ 0.04[c] | 5.50 $\pm$ 0.15[g] | 0.12 $\pm$ 0.01[c] |
| CR | 11.14 $\pm$ 0.25[d] | 0.20 $\pm$ 0.01[ab] | 0.60 $\pm$ 0.17[ab] | - |
| DCH | - | - | - | - |

Continuation of table 3

| Wheat-Grass | | | | |
|---|---|---|---|---|
| Anthocyanins | Black | Purple | Blue | White |
| DGI | - | - | - | - |
| DG | - | - | - | - |
| DR | 2.03 $\pm$ 0.08[b] | 0.21 $\pm$ 0.03[b] | 2.36 $\pm$ 0.05[d] | - |
| CG | 4.74 $\pm$ 0.51[d] | 0.23 $\pm$ 0.02[b] | - | 0.37 $\pm$ 0.04[b] |
| PODG | - | - | - | 0.26 $\pm$ 0.04[a] |
| PTG | - | - | - | - |

(continued)

| Wheat-Grass | | | | |
|---|---|---|---|---|
| Anthocyanins | Black | Purple | Blue | White |
| PLG | $2.77 \pm 0.11^{c}$ | $0.41 \pm 0.03^{d}$ | $0.41 \pm 0.03^{d}$ | $0.35 \pm 0.02^{b}$ |
| POG | $1.03 \pm 0.15^{a}$ | $0.32 \pm 0.06^{c}$ | $0.21 \pm 0.08^{b}$ | - |
| POR | - | $0.18 \pm 0.03^{ab}$ | $0.21 \pm 0.08^{b}$ | - |
| MG | $0.12 \pm 0.01^{c}$ | $1.25 \pm 0.17^{a}$ | - | - |
| CR | - | - | - | - |
| DCH | - | $1.89 \pm 0.30^{b}$ | $0.21 \pm 0.08^{b}$ | |

Values are the means of three replicates $\pm$ standard deviation. Different letters in each column represent significantly different values (p < 0.05) designated as a < b < c. Similar letters in the column represent values being statistically on par. DGI-delphinidin-3-o-galactoside chloride; DG-delphinidin-3-o-glucoside chloride; DR-delphinidin-3-o-rutinoside chloride; CG-cyanindin-3-o-glucoside chloride; PODG-peonidin-3,5-di-o-glucoside chloride; PTG-petunidin-3-o-glucoside chloride; PLG-pelargonidin 3-o-glucoside chloride; POG-peonidin-3-o-glucoside chloride; POR-peonidin-3-o-rutinoside chloride; MG-malvidin-3-o-glucoside chloride; CR-cyanindin-3-o-rutinoside chloride; DCH-delphin chloride (delphinidin-3,5-di-o-glucoside chloride). The blue wheat flour anthocyanin extract chromatograph consisted of 10 identified anthocyanin peaks similar to that of black wheat flour extracts, except it did not contain peonidin-3-o-glucoside chloride. In the case of blue wheat extracts, the highest concentration was observed in the case of malvidin-3-o-glucoside chloride (5.5 ppm), followed by delphinidin-3-o-galactoside chloride (4.95 ppm), cyanindin-3-o-glucoside chloride (4.5 ppm), and petunidin-3-o-glucoside chloride (3.18 ppm).

Chromatography provides the folowing results. Purple wheat flour anthocyanin extract chromatographs showed the presence of six identified anthocyanin peaks, with the highest concentration observed for cyanindin-3-o-glucoside chloride (2.64 ppm) followed by pelargonidin 3-o-glucoside chloride (1.88 ppm) and malvidin-3-o-glucoside chloride (1.32 ppm). The other peaks were identified as delphinidin-3-o-galactoside chloride, delphinidin-3-o-glucoside chloride, and cyanindin-3-o-rutinoside chloride.

The results revealed high antimicrobial activity of anthocyanin extracts from colored wheat flour and wheat-grass juice against the human pathogens in comparison to white wheat. The antimicrobial activity of the extracts was dose-dependent. Black wheat flour anthocyanin extracts (50-200 mg/mL) showed antimicrobial activity against all the pathogens. Among wheat flour anthocyanin extracts, antimicrobial activity followed the order black wheat > purple wheat > blue wheat > white wheat. All extracts produced clear halo zones against S *aureus, P. aeruginosa,* and *C. albicans* . Black wheat flour extracts (200 mg/mL) showed significantly higher zones of inhibition against *C. albicans* (2.57 cm) and *S aureus* (2.50 cm) amongst all flour extracts. Black and purple wheat flour extracts (200 mg/mL) showed maximum but statistically similar zones of inhibition against *P. aeruginosa* (2.73 and 2.60 cm) and *E. coli* (2.50 and 2.37 cm) in comparison to other flour extracts

Plant material included three colored wheat varieties of *Triticum aestivum viz.,* black wheat (NABIMG-11), purple wheat (NABIMG-10), blue wheat (NABIMG-9), and one white wheat (PBW621). All the colored wheat varieties were developed at the National Agri-Food Biotechnology Institute (NABI), Mohali, Punjab, India through breeding techniques for anthocyanin biofortification using donor colored wheat lines and recipient white wheat variety.

The provided results shows that the black wheat has more anthocyanin content and potential as regards other types, followed by purple. That explains the use of common wheat variety with black grain (Chornobrova) in breeding processes for obtaining the spelt variety Bilberry.

However, as discussed above, the wheat is understood as being allergic agent that causes the necessity to find more tolerant cereal varieties. Spelt has proved the better results in this respect over the years. One more factor is consider that black grain cereals normally provide not purely black, but browny-grey colour flour which is not attractive for consumers. From other side, purple grain varieties provide better appearance with reaching final black colour of grain, flour and products therefrom, that explains a choice of Schwabenkorn as a donor of purple grain.

The plant Bilberry obtained by such way has a purple grain and following anthocyanin content, as shown in the Table 4, Mg\kg:

Table 4

| Parent Schwabenkorn | Parent Chornobrova | Bilberry |
|---|---|---|
| 5 | 50 | 60-80 |

The measurement of anthocyanin content has been done by the following method.

The total anthocyanin content was determined according to: Abdel-Aal E.-S. M. and Hucl P. A Rapid Method for quantifying Total Anthocyanins in Blue Aleurone and Purple Pericarp Wheats. Cereal Chem. 76(3):350-354, 1999.

A ground wheat sample (3 g) was weighed in a 50-mL centrifuge tube, and 24 mL of acidified ethanol (ethanol and HCl 1.0N, 85:15, v/v) was added. The solution was mixed and adjusted to pH 1 with 4N HCl. The resulting solution was shaken for 15 min, readjusted to pH 1 if necessary, and the solution was shaken for an additional 15 min. The tube was centrifuged at 27,200 $\times$ g for 15 min, and the supernatant was poured into a 50-mL volumetric flask and made up to volume with acidified ethanol. Absorbance was measured at 535 nm against a reagent blank. Cyanidin 3-glucoside or Kuromanin from Extrasynthese (Genay, France) was used as a standard pigment. A series of cyanidin 3-glucoside standard solutions was prepared at 0-0.02 mmol (0-27 µg/3 mL). Absorbance was read at 535 nm against a reagent blank. The concentrations showed a linear relationship against absorbance and had regression and determination coefficients of 0.0197 and 0.999, respectively. Total anthocyanin content per sample (mg/kg) was calculated as cyanidin 3-glucoside:

$$C = (A/\varepsilon) \times (vol/1{,}000) \times MW \times (1/\text{sample wt}) \times 10^{6}$$

where C is concentration of total anthocyanin (mg/kg), A is absorbance reading, $\varepsilon$ is molar absorptivity (cyanidin 3-glucoside = 25,965 cm-1 M-1), Vol is total volume of anthocyanin extract, and MW is molecular weight of cyanidin 3-glucoside = 449.

The total anthocyanin content and antioxidant potential in colored wheat flour and wheat-grass juice extracts were significantly higher than white flour and wheat-grass juice extracts. Ultra-performance liquid chromatography showed the maximum number of anthocyanin peaks in black wheat, with delphinidin-3-o-galactoside chloride, delphinidin-3-o-glucoside chloride, and cyanindin-3-o-glucoside chloride as the major contributors. Among flour extracts, maximum zones of inhibition against *Staphylococcus aureus* (MTCC 1934), *Pseudomonas aeruginosa* (MTCC 1434), *Escherichia coli,* and *Candida albicans* (MTCC 227) were produced by black flour extract, having the highest anthocyanin content. It exhibited a minimum microbicidal concentration (MMC) of 200 mg/mL against *E. coli and C. albicans*; and 100 and 150 mg/mL against *S aureus* and *P. aeruginosa,* respectively. Black and purple flour extracts exhibited a minimum inhibitory concentration (MIC) of 50 mg/mL against *S aureus* and *P. aeruginosa.* White flour extracts did not show mmC against *E. coli* and *C. albicans.* Among wheat-grass juice extracts, black wheat-grass was most effective and showed an MIC of 100-150 mg/mL against all pathogens. It exhibited an mmC of 200 mg/mL against *S aureus* and *P. aeruginosa.* Hence, anthocyanin-rich colored wheat could be of nutraceutical importance.

Spelt variety BILBERRY has shown uniformity and stability for all traits, as described in the variety description information provided herein. It has been self- pollinated a sufficient number of generations, with careful attention to uniformity of plant type to ensure homozygosity and phenotypic stability. The line has been increased with continued observation for uniformity. No variant traits have been observed or are expected in BILBERRY, as described, for example, in Table 5.

Table 5

| Bilberry variety, % | 2021 | 2022 | 2023 |
|---|---|---|---|
| Field trials in dryland Odesa region, Ukraine | 3.3 | 4.0 | 3.0 |

Field crops are bred through techniques that take advantage of the plant's method of pollination, such as self-pollination, sib-pollination or cross- pollination. As used herein, the term cross-pollination includes pollination with pollen from a flower on a different plant from a different family or line and does not include self- pollination or sib- pollination. Spelt plants (Triticum spelta L.), are recognized to be naturally self- pollinated plants which, while capable of undergoing cross-pollination, rarely do so in nature. Thus intervention for control of pollination is needed for the establishment of superior varieties. Provided are methods of producing progeny with a new combination of genetic traits by cross pollinating one spelt plant with another by emasculating flowers of a designated female plant and pollinating the female parent with pollen from the designated male parent. Suitable methods of cross-pollination of plants are described, for example, in U.S. Patent No. 8,809,654, but other methods can be used, or modified, as is known to those skilled in the art. A cross between two different homozygous lines produces a uniform population of hybrid plants that may be heterozygous for many gene loci. A cross of two heterozygous plants each that differ at a number of gene loci will.produce a population of plants that differ genetically and will not be uniform. Regardless of parentage, plants that have been self-pollinated and selected for type for many generations become homozygous at almost all gene loci and produce a uniform population of true breeding progeny. The term "homozygous plant" is hereby defined as a plant with homozygous genes at 95% or more of its loci. Choice of breeding or selection methods depends on the mode of plant reproduction, the heritability of the trait(s) being improved, and the type of variety used commercially (e.g., F1 hybrid variety, pureline variety, etc.). For highly heritable traits, a choice of superior individual plants evaluated at a single location will be effective, whereas for traits with low heritability, selection can be based on mean values obtained from replicated evaluations of families of related plants. Popular selection methods which can be used include pedigree selection, modified pedigree selection, mass selection, and recurrent selection. The complexity of inheritance influences choice of the breeding method. For example, pedigree breeding, backcross breeding, single seed descent, and bulk breeding, which are each described in U.S. Patent No. 8,809,654, can be used. Each breeding program may include a periodic, objective evaluation of the efficiency of the breeding procedure. Evaluation criteria vary depending on the goal and objectives, but may include gain from selection per year based on comparisons to an appropriate standard, overall value of the advanced breeding lines, and number of successful varieties produced per unit of input (e.g., per year, per dollar expended, etc.). Various recurrent selection techniques can be used to improve quantitatively inherited traits controlled by numerous genes. The use of recurrent selection in self- pollinating crops depends on the ease of pollination and the number of hybrid offspring from each successful cross. Recurrent selection can be used to improve populations of either self- or cross-pollinated crops. A genetically variable population of heterozygous individuals is either identified or created by intercrossing several different parents. The best plants are selected based on individual superiority, outstanding progeny, or excellent combining ability. The selected plants are intercrossed to produce a new population in which further cycles of selection are continued. Plants from the populations can be selected and selfed to create new varieties. Spelt variety BILBERRY can be used as the female or the male parent in biparental crosses in order to develop new and valuable spelt varieties or hybrids. Spelt normally self-pollinates in nature. Cross pollination of one spelt plant with another to produce progeny with a new combination of genetic traits, can be carried out according to methods known to those skilled in the art. Spelt cross-pollination is achieved by emasculating flowers of a designated female plant and pollinating the female parent with pollen from the designated male parent. Methods of cross-pollinating plants for use in selection and advancement are described, for example in US Patent No. 9,282,712. Plant breeding methods may include analysis , comparison and characterization of the plant genome and the use of molecular markers, including techniques such as Starch Gel Electrophoresis, Isozyme Electrophoresis, Restriction Fragment Length Polymorphisms (RFLPs), Randomly Amplified Polymorphic DNAs (RAPDs), Arbitrarily Primed Polymerase Chain Reaction (AP-PCR), DNA Amplification Fingerprinting (DAF), Sequence Characterized Amplified Regions (SCARS), Amplified Fragment Length Polymorphisms (AFLPs), Simple Sequence Repeats (SSRs), Single Nucleotide Polymorphisms (SNPs) and Quantitative Trait Loci (QTL) mapping. Molecular markers can also be used during the breeding process for the selection of qualitative traits. For example, markers closely linked to alleles or markers containing sequences within the actual alleles of interest can be used to select plants that contain the alleles of interest during a crossing or backcrossing breeding program. The markers can also be used to select for the genome of the recurrent parent and against the markers of the donor parent. Using this procedure can minimize the amount of genome from the donor parent that remains in the selected plants. It can also be used to reduce the number of crosses back to the recurrent parent needed in a backcrossing program. The production of double haploids can also be used for the development of homozygous lines in the breeding program and in the production of, for example, hybrid spelt using variety BILBERRY. Double haploids are produced by the doubling of a set of chromosomes (1N) from a heterozygous plant to produce a completely homozygous individual. This can be advantageous because the process omits the generations of selfing needed to obtain a homozygous plant from a heterozygous source. Hybrid spelt can be produced, for example, in methods utilizing cytoplasmic male sterility, nuclear genetic male sterility, chemicals, genetic modification or a combination thereof. Spelt variety BILBERRY can be crossed with one or more parental lines, followed by repeated selfing and selection, producing many new genetic combinations. Selected germplasm can be grown under unique and different geographical, climatic and soil conditions with further selections being made during and at the end of the growing season. Spelt varieties that are highly homogeneous, homozygous and reproducible are useful as commercial varieties. There are many analytical methods, such as those described herein, which can be used to determine the homozygotic stability, phenotypic stability, and identity of these varieties produced or derived from variety BILBERRY. Gel electrophoresis is particularly useful. Spelt variety identification can occur, for example, through electrophoresis of gliadin, glutenin, albumin and globulin, and total protein extracts. In one of the embodiments of the present invention, electrophoresis of protein has been used as well as sedimentation method SDS-30K.

Disclosed are plant breeding methods in which plant populations as well as individual plants are evaluated for general health, agronomics, and stability at one or more stages. These evaluations can include, but are not limited to, one or more of the following characteristics: plant architecture traits such as seedling coleoptile length, coleoptile color (presence of anthocyanin), juvenile plant growth habit, tillering, plant height, straw strength or lodging, flag leaf carriage at boot stage, leaf width and length, glaucosity of stems, leaves and spikes, pubescence of leaves and spikes, spike shape, spike density, spike awnedness, and plant color through-out stages of growth; plant growth characteristics, such as vernalization requirement, date for first stem joint emergence, heading date, flowering date, physiological maturity date and harvest maturity; tolerance to weather conditions, such as cold tolerance, resistance to heaving, tolerance to wet soils and standing water, drought and heat tolerance; and grain characteristics, such as grain yield, test weight, 1000 kernel weight, grain moisture, grain color, grain shape, grain protein, flour milling yield and baking characteristics. During its development, spelt variety BILBERRY was assayed and/or planted in field trials and evaluated for a variety of traits and/or characteristics as compared to check varieties. The property(s) of appropriate check varieties include but are not limited to varieties with a similar relative maturity, varieties known to be susceptible to one or more particular diseases, insect, pathogen, field condition, weather condition, soil type or condition, and/or crop management practice, varieties known to be tolerant or resistant to one or more particular diseases, insect, pathogen, field condition, weather condition, soil type or condition, and/or crop management practice, varieties comprising one or more particular marker locus, and/or varieties derived from another appropriate variety or having a particular pedigree. Appropriate choice of check varieties for comparison assures an appropriate baseline and valid qualitative or quantitative assessment of any test varieties. In the development of BILBERRY, the plants can be tested for various traits including, but not limited to grain yield, test weight, heading date, harvest maturity, plant height, straw strength, pre-harvest sprout tolerance, resistance levels to leaf rust, stripe rust, tan spot, Septoria tritici blotch, Stagnospora nodorum blotch, powdery mildew, Fusarium (scab), wheat yellow mosaic virus and soilborne mosaic virus, and grain characteristics such as flour yield, flour protein, and baking characteristics. Spelt variety BILBERRY, being substantially homozygous, can be reproduced by planting seeds of the line, growing the resulting spelt plants under self- pollinating or sib-pollinating conditions, and harvesting the resulting seed, using techniques familiar to the agricultural arts.

In one aspect, spelt plants, plant parts and seeds are provided which have all or essentially all of the characteristics set forth in Table 1. In one aspect spelt plants, plant parts and seeds are provided which have all or essentially all of the physiological and morphological characteristics of spelt variety BILBERRY, or all or essentially all of the phenotypic characteristics of spelt variety BILBERRY, representative seed having been deposited with the depositarium as disclosed herein. Spelt variety BILBERRY can be further reproduced by tissue culture and regeneration. Tissue culture of various tissues of Triticum varieties and regeneration of plants therefrom is well known and widely published. Thus, in another aspect provided to are cells which upon growth and differentiation produce spelt plants capable of having the physiological and morphological characteristics of spelt variety BILBERRY. As used herein, the term "plant parts" includes, without limitation, plant protoplasts, plant cell tissue cultures from which spelt plants can be regenerated, plant calli, plant clumps, plant cells, embryos, pollen, ovules, pericarp, seed, flowers, florets, heads, spikes, stems, stalks, leaves, roots, root tips, anthers, and the like. When indicating that a plant is crossed or selfed this indicates that any plant part of the plant can be used. For instance the plant part does not need to be attached to the plant during the crossing or selfing, only the pollen might be used. In one aspect, a spelt plant containing a locus conversion or an essentially derived variety of BILBERRY is provided. Essentially derived varieties may be obtained, for example, by the selection of a natural or induced mutant, or of a somaclonal variant, the selection of a variant individual from plants of the initial variety, backcrossing, or transformation by genetic engineering, from the repeated use of variety BILBERRY or being predominately derived from variety BILBERRY. A locus conversion refers to plants within a variety that have been modified in a manner that retains the overall genetics of the variety and further comprises one or more loci with a specific desired trait, such as male sterility, insect, disease or herbicide resistance. Examples of single locus conversions include mutant genes, transgenes and native traits finely mapped to a single locus. One or more locus conversion traits may be introduced into a single spelt variety. Transgenes and transformation methods provide means to engineer the genome of plants to contain and express heterologous genetic elements, including but not limited to foreign genetic elements, additional copies of endogenous elements, and/or modified versions of native or endogenous genetic elements, in order to alter at least one trait of a plant in a specific manner. Any heterologous DNA sequence(s), whether from a different species or from the same species, which are inserted into the genome using transformation, backcrossing, or other methods known to one of skill in the art are referred to herein collectively as transgenes. The sequences are heterologous based on sequence source, location of integration, operably linked elements, or any combination thereof. One or more transgenes of interest can be introduced into spelt variety BILBERRY. In some examples, transgenic variants of spelt variety BILBERRY are produced by introducing at least one transgene of interest into spelt variety BILBERRY by transforming spelt variety BILBERRY with a polynucleotide comprising the transgene of interest. In other examples, transgenic variants of speltvariety BILBERRY are produced by introducing at least one transgene by introgressing the transgene into spelt variety BILBERRY by crossing. In one example, a process for modifying spelt variety BILBERRY with the addition of a desired trait, said process comprising transforming a spelt plant of spelt variety BILBERRY with a transgene that confers a desired trait is provided. In other examples, the genome of spelt variety BILBERRY is transformed by genetic modification using techniques described herein, such as the CRISPR/Cas system adapted for use in plants. Therefore, transgenic spelt variety BILBERRY cells, plants, plant parts, and seeds produced from this process are provided. In some examples one or more desired traits may include traits such as herbicide resistance, insect resistance, disease resistance, decreased phytate, modified fatty acid profile, modified fatty acid content, carbohydrate metabolism, protein content, or oil content. Numerous methods for plant transformation are known in the art, including biological, such as the use of Agrobacteria, and physical, such as biolistic and particle bombardment, plant transformation protocols. In addition, expression vectors and in vitro culture methods for plant cell or tissue transformation and regeneration of plants such as those known in the art can be used. In general, methods to transform, modify, edit or alter plant endogenous genomic DNA include altering the plant native DNA sequence or a pre-existing transgenic sequence including regulatory elements, coding and non-coding sequences. These methods can be used, for example, to target nucleic acids to pre-engineered target recognition sequences in the genome. Such pre-engineered target sequences may be introduced by genetic transformation such as genome editing or modification. As an example, a genetically modified plant variety can be generated using "custom" or engineered endonucleases such as meganucleases produced to modify plant genomes (see e.g., WO 2009/114321; Gao et al. (2010) Plant Journal 1:176-187). Another site- directed engineering method is through the use of zinc finger domain recognition coupled with the restriction properties of restriction enzyme. See e.g., Urnov, et al., (2010) Nat Rev Genet. 11(9):636-46; Shukla, et al., (2009) Nature 459 (7245):437-41. A transcription activator-like (TAL) effector-DNA modifying enzyme (TALE or TALEN) is also used to engineer changes in plant genome. See e.g., US20110145940, Cermak et CA 2977088 2017-08-23 al., (2011) Nucleic Acids Res. 39(12) and Boch et al., (2009), Science 326(5959): 1509-12. Site-specific modification of plant genomes can also be performed using the bacterial type II CRISPR (clustered regularly interspaced short palindromic repeats)/Cas (CRISPR-associated) system. See e.g., Belhaj et al., (2013), Plant Methods 9: 39; The Cas9/guide RNA-based system allows targeted cleavage of genomic DNA guided by a customizable small noncoding RNA in plants (see e.g, WO 2015026883A1. Plant transformation methods may involve the construction of an expression vector. Such a vector or recombinant construct comprises a DNA sequence that contains a coding sequence, such as a protein and/or RNA coding sequence under the control of or operatively linked to a regulatory element, for example a promoter. The vector or construct may contain one or more coding sequences and one or more regulatory elements. A genetic trait which has been engineered into the genome of a particular spelt plant may then be moved into the genome of another variety using traditional breeding techniques that are well known in the plant breeding arts. For example, a backcrossing approach is commonly used to move a transgene from a transformed spelt variety into an elite spelt variety, and the resulting backcross conversion plant would then contain the transgene(s). Various genetic elements can be introduced into the plant genome using transformation. These elements include, but are not limited to genes; coding sequences; inducible, constitutive, and tissue specific promoters; enhancing sequences; and signal and targeting sequences. Provided are plants genetically engineered or transformed to express various phenotypes of agronomic interest. Expression of genes can be altered to enhance disease resistance, insect resistance, herbicide resistance, agronomic, grain quality, and other traits relative to a comparable spelt plant that does not contain the transformed element or to a comparable non-transformed plant. Transformation can also be used to insert DNA sequences which control or help control male- sterility. DNA sequences native to wheat as well as non-native DNA sequences can be transformed into the spelt plants described herein and used to alter levels of native or non-native proteins. Various promoters, targeting sequences, enhancing sequences, and other DNA sequences can be inserted into the genome for the purpose of altering the expression of proteins. Reduction or increase in the activity of specific genes by genetic transformation or modification can effect gene silencing, gene suppression or gene over expression in the plants described herein. Many techniques for gene silencing are well known to one of skill in the art, including but not limited to, knock-outs, such as by insertion of a transposable element, antisense technology, (see U.S. Patents 5,107,065; 5,453,566; and 5,759,829), co- suppression, RNA interference, virus-induced gene silencing, hairpin structures, ribozymes, oligonucleotide-mediated targeted modification (see, e.g., WO03/076574 and WO99/25853), Zn-finger targeted molecules (see, e.g., WO01/52620; WO03/048345; and WO00/42219), use of exogenously applied RNA (see, e.g., US20110296556), and other methods known to those of skill in the art or combinations of the above methods. A genetic trait, engineered into a spelt plant using transformation techniques can be transferred into another line using traditional breeding techniques that are well known in the plant breeding arts. The spelt plants described herein can be the donor or the recipient of the transformed genetic trait. For example, a backcrossing approach can be used to move a transgene from a transformed spelt plant to an elite wheat variety to provide resulting progeny comprising a transgene. As used herein, "crossing" can refer to a simple X by Y cross, or the process of backcrossing, depending on the context. The term "breeding cross" excludes the processes of selfing or sibbing. Transgenic or genetically modified spelt plants described herein can be harvested to produce a foreign or modified protein in commercial quantities. The foreign or modified protein can be extracted from a tissue of interest, such as a seed, or from total biomass by known methods. The approximate chromosomal location of the integrated or modified DNA molecule can be determined from a genetic map generated, for example, via conventional RFLP, PCR, and SSR analysis. Particular markers used for these purposes may include any type of marker and marker profile which provides a means of distinguishing varieties. A genetic marker profile can be used, for example, to identify plants of the same variety or related varieties or to determine or validate a pedigree. In addition to being used for identification of spelt variety BILBERRY and its plant parts, the genetic marker profile is also useful in developing a locus conversion of variety BILBERRY. Methods of isolating nucleic acids from spelt plants and methods for performing genetic marker profiles using SNP and SSR polymorphisms are well known in the art. SNPs are genetic markers based on a polymorphism in a single nucleotide. A marker system based on SNPs can be highly informative in linkage analysis relative to other marker systems in that multiple alleles may be present. Methods for analyzing polynucleotides from plants, plant parts or seeds described herein may include contacting a polynucleotide from the plant, plant part or seed, such as from spelt variety BILBERRY with a molecular marker or with modified nucleotides that facilitate sequencing of the polynucleotide. The polynucleotide may be isolated, separated or otherwise obtained from the plant, plant part or seed. Modified nucleotides such as dNTPs may be incorporated with the polynucleotides along with appropriate primers in a reaction mixture that facilitates sequencing.

Sequencing can be done using any method known in the art. A method comprising isolating nucleic acids, such as DNA, from a plant, a plant part, plant cell or a seed of the wheat varieties disclosed herein is provided. The method can include mechanical, electrical and/or chemical disruption of the plant, plant part, plant cell or seed, contacting the disrupted plant, plant part, plant cell or seed with a buffer or solvent, to produce a solution or suspension comprising nucleic acids, optionally contacting the nucleic acids with a precipiting agent to precipitate the nucleic acids, optionally extracting the nucleic acids, and optionally separating the nucleic acids such as by centrifugation or by binding to beads or a column, with subsequent elution, or a combination thereof. If DNA is being isolated, an RNase can be included in one or more of the method steps. The nucleic acids isolated can comprise all or substantially all of the genomic DNA sequence, all or substantially all of the chromosomal DNA sequence or all or substantially all of the coding sequences (cDNA) of the plant, plant part, or plant cell from which they were isolated. The nucleic acids isolated can comprise all, substantially all, or essentially all of the genetic complement of the plant. The nucleic acids isolated can comprise a genetic complement of the spelt variety. The amount and type of nucleic acids isolated may be sufficient to permit whole genome 23 CA 2977088 2017-08-23 sequencing of the plant from which they were isolated or chromosomal marker analysis of the plant from which they were isolated. The methods can be used to produce nucleic acids from the plant, plant part, seed or cell, which nucleic acids can be, for example, analyzed to produce data. The data can be recorded. The nucleic acids from the disrupted cell, the disrupted plant, plant part, plant cell or seed or the nucleic acids following isolation or separation can be contacted with primers and nucleotide bases, and/or a polymerase to facilitate PCR sequencing or marker analysis of the nucleic acids. In some examples, the nucleic acids produced can be sequenced or contacted with markers to produce a genetic profile, a molecular profile, a marker profile, a haplotype, or any combination thereof. In some examples, the genetic profile or nucleotide sequence is recorded on a computer readable medium. In other examples, the methods may further comprise using the nucleic acids produced from plants, plant parts, plant cells or seeds in a plant breeding program, for example in making crosses, selection and/or advancement decisions in a breeding program. Crossing includes any type of plant breeding crossing method, including but not limited to crosses to produce hybrids, outcrossing, selfing, backcrossing, locus conversion, introgression and the like. Favorable genotypes and or marker profiles, optionally associated with a trait of interest, may be identified by one or more methodologies. In some examples one or more markers are used, including but not limited to AFLPs, RFLPs, ASH, SSRs, SNPs, indels, padlock probes, molecular inversion probes, microarrays, sequencing, and the like. In some methods, a target nucleic acid is amplified prior to hybridization with a probe. In other cases, the target nucleic acid is not amplified prior to hybridization, such as methods using molecular inversion probes (see, for example Hardenbol et al. (2003) Nat Biotech 21 :673-678). In some examples, the genotype related to a specific trait is monitored, while in other examples, a genome-wide evaluation including but not limited to one or more of marker panels, library screens, association studies, microarrays, gene chips, expression studies, or sequencing such as whole-genome resequencing and genotyping-by-sequencing (GBS) may be used. In some examples, no target- specific probe is needed, for example by using sequencing technologies, including but not limited to next-generation sequencing methods (see, for example, Metzker (2010) Nat 24 CA 2977088 2017-08-23 Rev Genet 11:31-46; and, Egan et al. (2012) Am J Bot 99:175-185) such as sequencing by synthesis (e.g., Roche 454 pyrosequencing, Illumina Genome Analyzer, and Ion Torrent PGM or Proton systems), sequencing by ligation (e.g., SOLiD from Applied Biosystems, and Polnator system from Azco Biotech), and single molecule sequencing (SMS or third-generation sequencing) which eliminate template amplification (e.g., Helicos system, and PacBio RS system from Pacific BioSciences). Further technologies include optical sequencing systems (e.g., Starlight from Life Technologies), and nanopore sequencing (e.g., GridION from Oxford Nanopore Technologies). Each of these may be coupled with one or more enrichment strategies for organellar or nuclear genomes in order to reduce the complexity of the genome under investigation via PCR, hybridization, restriction enzyme (see, e.g., Elshire et al. (2011) PLoS ONE 6:e19379), and expression methods. In some examples, no reference genome sequence is needed in order to complete the analysis. Variety BILBERRY and its plant parts can be identified through a molecular marker profile. Such plant parts may be either diploid or haploid. As described herein, genes or coding sequences can be expressed in transformed plants. More particularly, plants can be genetically engineered to express various phenotypes of agronomic interest. A single gene or locus conversion or at least about 1, 2, 3, 4, 5, 6, 7, 8, 9, 10, 11, 12, 13, 14, 15, 16, 17, 18, 19, 20, 25, 30, 35 or 40 or more genes or locus conversions and less than about 100, 90, 80, 70, 60, 50, 40, 30, 20, 15, or 10 genes or locus conversions may be introduced into a plant or comprised in the genome of the spelt plant. Combinations or stacks of two or more genes or coding sequences described herein can be used. Through the transformation of wheat the expression of genes can be modulated to enhance disease resistance, insect resistance, herbicide resistance, water stress tolerance and agronomic traits as well as grain quality traits. These traits and the genes and organisms which may be targets are described in US Patent No. 8,809,554. Transformation can also be used to insert or modify DNA sequences which control or alter male-sterility. DNA sequences native to wheat can be modified as well as native and non-native DNA sequences can be introduced into wheat and used to modulate levels of native or non-native proteins. CA 2977088 2017-08-23 The sequences introduced can be heterologous comprising a coding sequence . operably linked to a heterologous regulatory element, such as a promoter. Exemplary genes which can be targeted include, but are not limited to, genes that confer resistance to pests such as Hessian fly, wheat stem sawfly, cereal leaf beetle, and/or green bug or disease, to pathogens *Cladosporium fulvum, Pseudomonas syringae, Fusarium graminearum* Schwabe, wheat rusts, *Septoria tritici, Septoria nodorum,* powdery mildew, *Helminthosporium* diseases, smuts, bunts, *Fusarium* diseases, bacterial diseases, and viral diseases. Other genes, coding sequences or targets which can be used include those encoding *Bacillus thuringiensis* protein, a derivative thereof or a synthetic polypeptide modeled thereon. Examples of *Bacillus thuringiensis* transgenes encoding a endotoxin and being genetically engineered are given in the following patents and patent applications: 5,188,960; 5,689,052; 5,880,275; 8,809,654; WO 91/14778; WO 99/31248; WO 01/12731; WO 99/24581; WO 97/40162 and US Patent Serial Nos. 7,605,304 and 7,696,412 and US Patent Publication Serial No. US2004/0091505. Other genes, coding sequences or targets which can be used include those encoding an insect-specific hormone or pheromone such as an ecdysteroid and juvenile hormone, a variant thereof, a mimetic based thereon, or an antagonist or agonist thereof, an insect diuretic hormone receptor, such as an allostatin (see also U.S. Patent No. 5,266,317), an enzyme responsible for a hyper accumulation of a monoterpene, a sesquiterpene, a steroid, hydroxamic acid, a phenylpropanoid derivative or another non- protein molecule with insecticidal activity, an enzyme involved in the modification, including the post-translational modification, of a biologically active molecule, for example, a glycolytic enzyme, a proteolytic enzyme, a lipolytic enzyme, a nuclease, a cyclase, a transaminase, an esterase, a hydrolase, a phosphatase, a kinase, a phosphorylase, a polymerase, an elastase, a chitinase and a glucanase, whether natural or synthetic; a molecule that stimulates signal transduction, for example mung bean calmodulin cDNA clones and maize calmodulin cDNA clones; a hydrophobic peptide (see US Patent Nos. 5,580,852 and US 5,607,914); a membrane permease, a channel former or a channel blocker, for example, cropin-beta lytic peptide analog conferring Pseudomonas solanacearum; an insect-specific antibody or an immunotoxin derived therefrom, or a virus-specific antibody; a developmental-arrestive protein such as a endopolygalacturonase-inhibiting protein or a ribosome-inactivating gene; genes involved in the Systemic Acquired Resistance (SAR) Response and/or the pathogenesis related genes, In some embodiments, coat protein-mediated resistance can be conferred in plants against one or more of alfalfa mosaic virus, cucumber mosaic virus, tobacco streak virus, potato virus X, potato virus Y, tobacco etch virus, tobacco rattle virus and tobacco mosaic virus. Such resistance may be conferred using, for example, a viral- invasive protein or a complex toxin derived therefrom. In some embodiments, genes, coding sequences or targets which can be used include, without limitation, antifungal genes (see, for example, US Publication No: 20020166141); detoxification genes, such as for fumonisin, beauvericin, moniliformin and zearalenone and their structurally related derivatives (see, for example, US Patent No. 5,792,931); cystatin and cysteine proteinase inhibitors (see for example, US Patent Publication Serial No: 20050102717), defensin genes (see for example, PCT Public WO03000863 and US Patent Publication Serial No: 20030041348); and genes conferring resistance to nematodes, see for example, WO 03/033651. Genes, coding sequences, or targets that confer resistance to a herbicide are described, for example, in U.S. Patent No. 8,809,654. Examples include genes or coding sequences encoding acetohydroxy acid synthase, a chimeric protein of rat cytochrome P4507A1, yeast NADPH-cytochrome P450 oxidoreductase, glutathione reductase, superoxide dismutase, phosphotransferases, ALS and AHAS enzymes and other genes or coding sequences which confer resistance to a herbicide such as an imidazalinone or a sulfonylurea (see also, U.S Patent Nos. 5,605,011; 5,013,659; 5,141,870; 5,767,361; 5,731,180; 5,304,732; 4,761,373; 5,331,107; 5,928,937; and 5,378,824; and international publication WO 96/33270); Glyphosate or glufosinate resistance can also be conferred using, for example, sequences encoding mutant 5- enolpyruv1-3-phosphikimate synthase (EPSP), aroA genes, phosphinothricin acetyl transferase (PAT), glyphosate oxido-reductase enzyme, glyphosate N- acetyltransferase, glutamine synthetase, Streptomyces hygroscopicus phosphinothricin acetyl transferase (bar) genes), and pyridinoxy or phenoxy proprionic acids and cycloshexones (ACCase inhibitor-encoding genes). See, for example, U.S. Patent Nos. 4,769,061, 4,975,374, 4,940,835, 5,776,760, 5,463,175, 5,627,061, 6,566,587; 6,338,961; 6,248,876 B1; 6,040,497; 5,804,425; 5,633,435; 5,145,783; 4,971,908; 5,312,910; 5,188,642; 4,940,835; 5,866,775; 6,225,114 B1; 6,130,366; 5,310,667; 4,535,060; 4,769,061; 5,633,448; 5,510,471; Re. 36,449; RE 37,287 E; and 5,491,288; US Patent Publication No. 20040082770 and international publications EP1173580; WO 01/66704; EP1173581 and EP1173582, EP 0 242 246 and EP 0 242236. See also, U.S. Patent Nos. 5,969,213; 5,489,520; 5,550,318; 5,874,265; 5,919,675; 5,561,236; 5,648,477; 5,646,024; 6,177,616; and 5,879,903. Triazine resistance can be conferred using, for example, psbA and gs+ genes, sequences encoding a benzonitrile (nitrilase gene) such as disclosed in U.S. Patent No. 4,810,648. Resistance to herbicides which target Protoporphyrinogen oxidase (protox) can also be conferred such resistance being described in U.S. Patent Nos. 6,288,306, 6,282,837, 5,767,373 and international publication WO 01/12825. Genes, coding sequences, or targets that confer or improve grain quality include, without limitation, altered fatty acids (for example, oleic, linoleic, linolenic), altered phosphorus content (for example, using phytase), altered carbohydrates such as modulating the branching pattern of starch or altering thioredoxin, *Bacillus subtilis levansucrase* gene, *Bacillus licheniformis alpha-amylase,* tomato invertase, alpha- amylase gene, starch branching enzyme II, UDP-D-xylose 4-epimerase, Fragile 1 and 2, Ref1, HCHL, C4H, high oil seed such as by modification of starch levels (AGP). Fatty acid modification genes mentioned above may also be used to affect starch content and/or composition through the interrelationship of the starch and oil pathways, altered content or composition of antioxidants such as tocopherol or tocotrienols, such as using a phytl prenyl transferase (ppt), or through alteration of a homogentisate geranyl geranyl transferase (hggt). Genes, coding sequences, or targets that can be targets to confer or improve grain quality are disclosed in, for example, see U.S. Pat. Nos. 8,809,654, 6,787,683, 6,531,648, 6,423,886, 6,232,529, 6,197,561, 6,825,397, US Patent Publication Nos. 2003/0079247, US2003/0204870, US2004/0034886 international PCT publications WO 02/42424, WO 98/22604, WO 03/011015, WO02/057439, WO03/011015, WO 99/10498, WO 00/68393, and WO 03/082899. Genes, coding sequences or targets for altered essential seed amino acids, such as one or more of lysine, methionine, threonine, tryptophan or altered sulfur amino acid content are also provided, can be used in the methods and plants described herein and are described in, for example, US Patent Nos. 8,809,654, 6803498, 6127600, 6,194,638, 6346403, 6080913, 5990389, 5939599, 5912414, 5850016, 5885802, 5885801, 5633436, 5559223, 6664445, 6459019, 6,194,638, 6,399,859, 6441274, international PCT applications WO99/40209, WO99/29882, WO98/20133, WO96/01905, WO98/56935, WO98/45458, WO98/42831, WO95/15392, WO01/79516, WO00/09706, and US Publication Nos.US2003/0150014, US2003/0163838, US2004/0068767, and U52004/0025203. Genes, coding sequences or targets that control or alter male sterility and methods for conferring male sterility and male sterile plants are provided. There are several methods of conferring genetic male sterility available, such as disclosed in U.S. Patents 8,809,654, 4,654,465 and 4,727,219, 3,861,709, 3,710,511, 5,432,068. For additional examples of nuclear male and female sterility systems and genes, see also, US 5,859,341; US 6,297,426; US 5,478,369; US 5,824,524; US 5,850,014; and US 6,265,640. Genes, coding sequences or targets that create a site for site specific DNA integration can also be used such as the introduction of FRT sites that may be used in the FLP/FRT system and/or Lox sites that may be used in the Cre/Loxp system. Other systems that may be used include the Gin recombinase of phage Mu, the Pin recombinase of E. coli, and the R/RS system of the pSR1 plasmid. Genes that affect abiotic stress resistance (including but not limited to flowering, ear and seed development, enhancement of nitrogen utilization efficiency, altered nitrogen responsiveness, drought resistance or tolerance, cold resistance or tolerance, and salt resistance or tolerance) and increased yield under stress are provided. For example, see: US Patent Nos. 8,809,654, 5,892,009, 5,965,705, 5,929,305, 5,891,859, 6,417,428, 6,664,446, 6,706,866, 6,717,034, 6,801,104, 6,177,275, 6,107,547, 6,084,153, US Patent Publication Nos. 2004/0148654, 2004/0237147, 2003/0166197, 29 CA 2977088 2017-08-23 2004/0128719, 2004/0098764, 2004/0078852, international PCT application WO2000060089, WO2001026459, WO2001035725, WO 00/73475; WO2001034726, WO2001035727, WO2001036444, WO2001036597, WO2001036598, WO2002015675, WO2002017430, WO2002077185, WO2002079403, WO2003013227, WO2003013228, WO2003014327, WO2004031349, WO2004076638, WO9809521, WO01/36596 and WO9938977, WO2000/006341, WO04/090143, WO0202776, WO2003052063, WO0164898, and WO200032761. Other genes and transcription factors that affect plant growth and agronomic traits such as yield, flowering, plant growth and/or plant structure, can be introduced or introgressed into plants, see e.g. WO97/49811 (LHY), WO98/56918 (ESD4), WO97/10339 and US6573430 (TFL), US6713663 (FT), WO96/14414 (CON), WO96/38560, WO01/21822 (VRN1), WO00/44918 (VRN2), WO99/49064 (GI), WO00/46358 (FRI), WO97/29123, U56794560, US6307126 (GAI), WO99/09174 (D8 and Rht), and WO2004076638 and WO2004031349 (transcription factors). Genes that confer agronomic enhancements, nutritional enhancements, or industrial enhancements can also be used. Such genes are described for example in US Patent No. 8,809,654. Such enhancements include, without limitation, improved tolerance to water stress from drought or high salt water condition. See e.g. US Patent Nos. 5,981,842, 5,780,709, international patent applications WO 92/19731, WO 92/19731. In some embodiments, methods of treating BILBERRY with a mutagen and the plant produced by mutagenesis of BILBERRY are provided. Backcross conversions of spelt variety BILBERRY are also described. A backcross conversion occurs when modified or non-native DNA sequences are introduced through traditional (non-transformation) breeding techniques, such as backcrossing. DNA sequences, whether naturally occurring, modified or transgenes, may be introduced using these traditional breeding techniques. Desired traits transferred through this process include, but are not limited to, nutritional enhancements, industrial enhancements, disease resistance, insect resistance, herbicide resistance, agronomic enhancements, grain quality enhancement, waxy starch, breeding enhancements, seed production enhancements, and male sterility. Descriptions of some of the cytoplasmic male sterility genes, nuclear male sterility genes, chemical hybridizing agents, male fertility restoration genes, and methods of using the aforementioned are discussed in "Hybrid Wheat by K.A. Lucken (pp. 444-452 In Wheat and Wheat Improvement, ed. Heyne, 1987). Examples of genes for other traits which can be used with the methods, plants and plant parts described herein include: Leaf rust resistance genes (Lr series such as Lr1, Lr10, Lr21, Lr22, Lr22a, Lr32, Lr37, Lr41, Lr42, and Lr43), Fusarium head blight-resistance genes (QFhs.ndsu-3B and QFhs.ndsu-2A), Powdery Mildew resistance genes (Pm21), common bunt resistance genes (Bt-10), and wheat streak mosaic virus resistance gene (Wsm1), Russian wheat aphid resistance genes (Dn series such as Dn1, Dn2, Dn4, Dn5), Black stem rust resistance genes (Sr38), Yellow rust resistance genes (Yr series such as Yr1, YrSD, Yrsu, Yr17, Yr15, YrH52), aluminum tolerance genes (Alt(BH)), dwarf genes (Rht), vernalization genes (Vrn), Hessian fly resistance genes (H9, H10, H21, H29), grain color genes (R/r), glyphosate resistance genes (EPSPS), glufosinate genes (bar, pat) and water stress tolerance genes (Hva1, mtID). The trait of interest is transferred from the donor parent to the recurrent parent, in this case, the spelt plant disclosed herein. Single gene traits, whether naturally occurring, induced by mutation or genetically altered, may result from either the transfer of a dominant allele or a recessive allele. Selection of progeny containing the trait of interest is done by direct selection for a trait associated with a dominant allele. Selection of progeny for a trait that is transferred via a recessive allele requires growing and selfing the first backcross to determine which plants carry the recessive alleles. Recessive traits may require additional progeny testing in successive backcross generations to determine the presence of the gene of interest. Methods of developing a backcross conversion BILBERRY spelt plant are provided including the step of repeated backcrossing spelt variety BILBERRY. The number of backcrosses made may be 2, 3, 4, 5, 6, 7, 8 or greater, and fewer than 50, 40, 30, 25, 20, 15, 10, 9, or 8. The specific number of backcrosses used will depend upon the genetics of the donor parent and whether molecular markers are utilized in the backcrossing program. Provided are plants and plant populations that are produced from backcrossing methods, transformation, locus conversion, or otherwise produced, and combinations thereof and that retain at least 70%, 75%, 79%, 80%, 81%, 82%, 31 CA 2977088 2017-08-23 83%, 84%, 85%, 86%, 87%, 88%, 89%, 90%, 91%, 92%, 93%, 94%, 95%, 96%, 97%, 98%, 99%, 99.5%, 99.6%, 99.7%, 99.8%, or 99.9% or 99.95%, 99.98%, 99.985%, 99.99% or 99.995% of the genetic profile of spelt variety BILBERRY. The percentage of the genetics retained in the backcross conversion may be measured by either pedigree analysis or through the use of genetic techniques such as molecular markers, or electrophoresis. Such methods and techniques are described in US Patent No. 8,809,654. The backcross conversion or locus conversion developed from this method may be similar to BILBERRY for the results listed in Tables 1, 2. Such similarity may be measured by a side by side phenotypic comparison, with differences and similarities determined at a 5% significance level, when appropriate in environmental conditions that account for the trait being transferred. For example, herbicide should not be applied in the phenotypic comparison of herbicide resistant backcross conversion of BILBERRY when compared back to BILBERRY. Described are methods for using spelt variety BILBERRY in plant breeding and plants and plant populations produced by such methods. For example, spelt variety BILBERRY can be crossed with another variety of wheat to form a first generation population of F1 plants. This first generation population of F1 plants will comprise an essentially complete set of the alleles of spelt variety BILBERRY. Also provided are methods and plants which use transgenic or backcross conversions of spelt variety BILBERRY to produce first generation F1 plants.

A method of developing a BILBERRY-progeny spelt plant comprising crossing BILBERRY with a second spelt plant and performing a breeding method is also described. An exemplary method for producing a line derived from spelt variety BILBERRY is as follows. Spelt variety BILBERRY is crossed with another variety of spelt, such as an elite variety. The F1 seed derived from this cross is grown to form a homogeneous population. The F1 seed contains one set of the alleles from variety BILBERRY and one set of the alleles from the other spelt variety. The F1 genome is 50% variety BILBERRY and 50% of the other elite variety. The F1 seed is grown and allowed to self, thereby forming F2 seed. On average the F2 seed would have derived 50% of its alleles from variety BILBERRY and 50% from the other wheat variety, but various individual plants from the population can have a much greater percentage of CA 2977088 2017-08-23 their alleles derived from BILBERRY. The F2 seed is grown and selection of plants made based on visual observation and/or measurement of traits. The BILBERRY- derived progeny that exhibit one or more of the desired BILBERRY-derived traits are selected and each plant is harvested separately. This F3 seed from each plant is grown in individual rows and allowed to self. Then selected rows or plants from the rows are harvested and threshed individually. The selections based on visual observation and/or measurements for desirable traits of the plants, such as one or more of the desirable BILBERRY-derived traits are made. The process of growing and selection is repeated any number of times until a homozygous BILBERRY-derived spelt plant is obtained. The homozygous BILBERRY-derived spelt plant contains desirable traits derived from spelt variety BILBERRY, some of which may not have been expressed by the other original wheat variety to which spelt variety BILBERRY was crossed and some of which may have been expressed by both wheat varieties but now would be at a level equal to or greater than the level expressed in spelt variety BILBERRY. The homozygous BILBERRY-derived spelt plants have, on average, 50% of their genes derived from spelt variety BILBERRY, but various individual plants from the population would have a much greater percentage of their alleles derived from BILBERRY. The breeding process, of crossing, selfing, and selection may be repeated to produce another population of BILBERRY-derived spelt plants with, on average, 25% of their genes derived from spelt variety BILBERRY, and with various individual plants from the population having a much greater percentage of their alleles derived from BILBERRY. Homozygous spelt variety BILBERRY derived spelt plants that have received BILBERRY-derived traits are also provided. In some instances, selection may or may not occur at every selfing generation, selection may occur before or after the actual self-pollination process occurs, or individual selections may be made by harvesting individual spikes, plants, rows or plots at any point during the breeding process described herein. In addition, double haploid breeding methods may be used at any step in the process. In one aspect, the population of plants produced at each and any generation of selfing, each such population consisting of plants containing approximately 50% of its genes from spelt variety BILBERRY, 25% of its genes from spelt variety BILBERRY in the second cycle of crossing, selfing, and selection, 12.5% of its genes from spelt variety BILBERRY in the third cycle of crossing, selfing, and selection, and so on. Also disclosed are methods of obtaining a homozygous BILBERRY-derived spelt plant by crossing spelt variety BILBERRY with another variety of wheat and applying double haploid methods to the F1 seed or F1 plant or to any generation of BILBERRY-derived wheat obtained by the selfing of this cross. Still further, methods for producing BILBERRY-derived spelt plants are provided by crossing spelt variety BILBERRY with a spelt plant and growing the progeny seed, and repeating the crossing or selfing along with the growing steps with the BILBERRY-derived spelt plant from 1 to 2 times, 1 to 3 times, 1 to 4 times, or 1 to 5 times. Thus, any and all methods using spelt variety BILBERRY in breeding, including selfing, pedigree breeding, backcrossing, hybrid production and crosses to populations are provided. Unique starch profiles, molecular marker profiles and/or breeding records can be used to identify the progeny lines or populations derived from these breeding methods. Also disclosed are methods of harvesting the grain of variety spelt variety BILBERRY and using the grain as seed for planting. Embodiments include cleaning the seed, treating the seed, and/or conditioning the seed. Cleaning the seed includes removing foreign debris such as weed seed and removing chaff, plant matter, from the seed. Conditioning the seed can include controlling the temperature and rate of dry down and storing seed in a controlled temperature environment. Seed treatment is the application of a composition to the seed such as a coating or powder. Seed material can be treated, typically surface treated, with a composition comprising combinations of chemical or biological herbicides, herbicide safeners, pesticides, insecticides, fungicides, nutrients, germination inhibitors, germination promoters, cytokinins, nutrients, plant growth regulators, antimicrobials, and activators, bactericides, nematicides, avicides, or molluscicides. These compounds are typically formulated together with further carriers, surfactants or application-promoting adjuvants customarily employed in the art of formulation. The coatings may be applied by impregnating propagation material with a liquid formulation or by coating with a combined wet or dry formulation. Examples of the various types of compounds that may be used as seed treatments are provided in The Pesticide Manual: A World Compendium, C.D.S. Tomlin Ed., published by the British Crop Production Council. Some specific seed treatments that may be used on crop seed include, but are not limited to, abscisic acid, acibenzolar-S-methyl, *avermectin, amitrol,* azaconazole, *azospirillum,* azoxystrobin, *bacillus, Bacillus subtilis, Bacillus simplex, Bacillus firmus, Bacillus amyloliquefaciens, Pasteuria genus* (e.g. *P. nishizawae*), *bradyrhizobium, captan, carboxin, chitosan, clothianidin,* copper, *cyazypyr, difenoconazole, etidiazole, fipronil, fludioxonil, fluquinconazole, flurazole, fluxofenim,* GB126, Harpin protein, imazalil, imidacloprid, ipconazole, isofavenoids, lipo-chitooligosaccharide, mancozeb, manganese, maneb, mefenoxam, metalaxyl, metconazole, PCNB, penflufen, penicillium, penthiopyrad, permethrine, picoxystrobin, prothioconazole, pyraclostrobin, rynaxypyr, S- metolachlor, saponin, sedaxane, TCMTB, tebuconazole, thiabendaxole, thiamethoxam, thiocarb, thiram, tolclofos-methyl, triadimenol, trichoderma, trifloxystrobin, triticonazole and/or zinc. Seed varieties and seeds with specific genetic resistance traits can be tested to determine which seed treatment options and application rates will complement such varieties and genetic resistance traits in order to enhance yield. For example, a variety with good yield potential but loose smut susceptibility will benefit from the use of a seed treatment that provides protection against loose smut. Likewise, a variety encompassing a genetic resistance trait conferring insect resistance will benefit from the second mode of action conferred by the seed treatment. Further, the good root establishment and early emergence that results from the proper use of a seed treatment will result in more efficient nitrogen use, a better ability to withstand drought and an overall increase in yield potential of a variety or varieties containing a certain trait when combined with a seed treatment. Spelt variety BILBERRY has traits and characteristics that distinguish it from other spelt t varieties. A description of the traits used to measure or characterize a spelt variety such as variety BILBERRY and the scoring ranges used for such traits are described below.

We were not aiming to develop the var. BILBERRY as a strictly identical copy of the original parental spelt-wheat Schwabenkorn. During the sequential steps of broken backcrossing by the pollen of the original spelt-wheat the short stem recombinant genotype (35-40 cm plant height lower if compared with the original spelt-wheat height) was isolated and used for the following backcrosses. As a result, the var. BILBERRY is substantially lower in plant height than the original Schwabenkorn. The lower plant height trait of the var. BILBERRY tightly linked with better tolerance to lodging if compared with original spelt. The short stem var. BILBERRY released better agronomic performance and tolerance to the spike breakage accompanying the loose of seeds and yield loss.

The major morphological character of var. BILBERRY differing it from any other spelt-wheat is its dark-purple grain pericarp caused by high concentration of polyphenolic pigments anthocyanins capable to increase considerably the grain antioxidant activity in comparison to original spelt-wheat. The higher grain antioxidant activity of the var. BILBERRY substantially enhances its food health-promoting functional property providing its considerable advantage as the valuable functional food in comparison with any other spelt-wheat with normal red grain color.

While sequential backcrossing steps performed the genotypes with maximal intensity of grain purple pericarp color trait were selected and used for the following backcrosses.

The var. BILBERRY has the spike thrashing ability character slightly better than it is appeared in the original spelt. Such an improved character provides better agronomic performance of the var. BILBERRY and its seed realizing.

The grain quality characters such as grain protein content, crude gluten content and its quality were not substantially different from the same characters of the original spelt-wheat.

In a preferred embodiment of the present invention the basic conception of the colored spelt wheat development was as follows. Since we have dealt with the original ancient virgin spelt wheat totally free of the modern breeding interference our target colored spelt wheat variety to be developed (our case Bilberry) a priory should be identical (ideal case) or tightly similar to the original one in the complex characters such as plant morphology, agronomic performance, tolerance to biotic and abiotic stresses, grain quality, etc.. The ancient spelt wheat status of the original spelt SchwabenKorn shouldn't be broken in the process of backcrossing events and target colored spelt wheat selection. Our final purpose was to develop target colored spelt wheat variety identical, or maximum tightly similar, to the original one with the only single difference - grain pericarp color.

Strictly according with this basic conception each breeding steps to be performed should be included comprehensive comparison of the selected target colored grain genotypes with original spelt SchwabenKorn taking into account the characters listed above.

The experimental field scheme was as follows: original spelt wheat plants and target genotypes selected were sowed in one-meter long rows with 0.45 m interval distance between rows. Each planting season at least 100 selected target genotypes with colored grain were subjected for evaluation and comparison with original spelt plants arranged as at least 40 one-meter long rows (approx. 800 plants). The seed drilling date and harvest date were the same as commonly accepted for the Odesa farming region (planting date 10-15 October, harvest date 10-20 July).

The final selected target colored grain elite spelt line (being named as Bilberry) had the maximum possible similarities in the complex characters with the original spelt excluding dark-purple grain color and reduced plant height.

After the seed propagation original spelt SchwabenKorn and target colored spelt selected were subjected for the field and laboratory experiments (2020-21 years). The field experiments were sowed as 6 square meter plots for both spelt wheats in 3 rept. The data of grain yield as well as agronomic performance for both spelt wheats were collected.

As long as two years field experimentation resulted in no difference found between both spelt wheats in grain yield level ranged as 25-30 q/ha. No difference was found in plant tolerance to diseases and drought stress. Just threshing ability and tolerance to lodging slightly better in target colored spelt was found. There was no difference found between both spelt wheats in milling and baking quality characters. Both spelt wheats expressed low baking quality with W dough elasticity as low as 75-80 alveograph units, P/L - 0.29, le% - 31.3.

It will be apparent to those of skill in the art that variations may be applied to the compositions and methods described herein and in the steps or in the sequence of steps of the method described herein without departing from the concept, spirit and scope of the invention. More specifically, it will be apparent that certain changes and modifications such as single gene conversions, including for example, modifications and mutations, somoclonal variants, variant individuals selected from large populations of the plants of the instant inbred and the like may be practiced. All such similar substitutes and modifications apparent to those skilled in the art are deemed to be within the spirit, scope and concept of the invention. It is to be understood that the invention is not limited in its application to the details of components set forth in the following description. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. It also is understood that any numerical range recited herein includes all values from the lower value to the upper value. For example, if a concentration range is stated as 1% to 50%, it is intended that values such as 2% to 40%, 10% to 30%, or 1% to 3%, etc., are expressly enumerated in this specification. These are only examples of what is specifically intended, and all possible combinations of numerical values between and including the lowest value and the highest value enumerated are to be considered to be expressly stated in this application. As used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

**Examples**

The following examples provide descriptions of several assays that can be used to characterize and/or select a spelt variety during one or more stages of variety development. Other methods and assays are available and can be used in combination with or instead of the examples provided herein.

Example 1: Flour Yield screening

The potential average flour yield of wheat can be determined on samples of grain that has been cleaned to standard and tempered to uniform moisture, using a test mill. Samples are milled to established parameters, the flour sifted into fractions, which are then weighed to calculate flour yield as a percentage of grain weight. Flour yield "as is" is calculated as the bran weight (over 40 weight) subtracted from the grain weight, divided by grain weight and times 100 to equal "as is" flour yield. Flour yield is calculated to a 15% grain moisture basis as follows: flour moisture is regressed to predict the grain moisture of the wheat when it went into the Quad Mill using the formula Initial grain moisture = 1.3429 X (flour moisture) ¨ 4. The flour yields are corrected back to 15% grain moisture after estimating the initial grain moisture using the formula Flour Yield(15%) = Flour Yield (as is) - 1.61% X (15% - Actual flour moisture)

Example 2: Flour protein screening. The protein content as a percentage of total flour may be estimated by the Kjeldahl method or properly calibrated near-infrared reflectance instruments to determine the total nitrogen content of the flour. Flour protein differences among cultivars can be a reliable indicator of genetic variation provided the varieties are grown together, but can vary from year to year at any given location. Flour protein from a single, non-composite sample may not be representative.

Example 3: Sucrose solvent retention capacity (SRC). The solvent retention capacity (SRC) of wheat flour measures the ability of the flour to retain various solvents after centrifugation. Sucrose SRC predicts the starch damage and pentosan components, and can be correlated to sugar-snap cookie diameter quality metrics. Sucrose SRC is a measure of arabinoxylans (also known as pentosans) content, which can strongly affect water absorption in baked products. Water soluble arabinoxylans are thought to be the fraction that most greatly increases sucrose SRC. Sucrose SRC a predictor of cookie quality, with sugar snap cookie diameters decreasing by 0.07 cm for each percentage point increase in sucrose SRC. The negative correlation between wire-cut cookie and sucrose SRC values is $r=-0.66$ ($p<0.0001$). Sucrose SRC typically increases in wheat samples with lower flour yield ($r=-0.31$) and lower softness equivalent ($r=-0.23$). The cross hydration of gliadins by sucrose also causes sucrose SRC values to be correlated to flour protein ($r=0.52$) and lactic acid SRC ($r=0.62$). Soft wheat flours for cookies typically have a target of 95% or less when used by the baking industry for biscuits and crackers. Sucrose SRC values increase by 1% for every 5% increase in lactic acid SRC. The 95% target value can be exceeded in flour samples where a higher lactic acid SRC is required for product manufacture since the higher sucrose SRC is due to gluten hydration and not to swelling of the water soluble arabinoxylans.

Example 4: Lactic acid SRC Lactic Acid SRC = Lactic Acid Solvent Retention Capacity. Lactic acid SRC measures gluten strength. Typical values are below 85% for "weak" soft varieties and above 105% or 110% for "strong" gluten soft varieties. See the above discussion of protein quality in this section for additional details of the lactic acid SRC. Lactic acid SRC results correlate to the SDS-sedimentation test. The lactic acid SRC is also correlated to flour protein concentration, but the effect is dependent on genotypes and growing conditions. The SWQL typically reports a protein-corrected lactic acid SRC value to remove some of the inherent protein fluctuation not due to cultivar genetics. Lactic acid is corrected to 9% protein using the assumption of a 7% increase in lactic acid SRC for every 1% increase in flour protein.

Example 5: Molecular screening As shown in Tables, plants were analyzed at various times throughout the development of BILBERRY for specific alleles for scab resistance. As discussed above, and as is known to those skilled in the art, other traits can also be screened by molecular analysis

Example 6: Performance of BILBERRY In this example, the traits and characteristics of spelt variety BILBERRY are given in paired comparisons with another variety during the same growing conditions and same year. The data collected on each variety is presented for a number of characteristics and traits (see Tables).

Example 7: Anthocyanin content according with the rapid method described by Abdel-Aal E.-S. M. and Hucl P. A. Rapid Method for quantifying Total Anthocyanins in Blue Aleurone and Purple Pericarp Wheats. Cereal Chem. 76(3):350-354, 1999.

Example 8: Breeding History, properties and adaptation of spelt variety BILBERRY Spelt variety BILBERRY is a spelt variety (Triticum spelta L.) Variety BILBERRY demonstrates excellent yield potential (average 3-4 t/ha) and test weight (770 g/l), excellent resistance to all most aggressive fungal diseases (mildew, leaf rust. stem rust, septoria and fusarium head blight) commonly spread in the South region of Ukraine. Var. Bilberry being short stem plant height (20-25 cm shorter compared to original spelt) is properly resistant to plant lodging and don't lose the spikes (grain yield) while harvest date is prolonged.

Spelt variety BILBERRY was developed by from a cross between four homozygous lines: variety Schwabenkorn (50%), variety Chornobrova (50%),Spelt variety BILBERRY, being substantially homozygous, can be reproduced by planting seeds of the line, growing the resulting spelt plants under self-pollinating or sib-pollinating conditions, and harvesting the resulting seed, using techniques familiar to the agricultural arts. The breeding history of BILBERRY is described below in Table 6.

Table 6: Breeding History of spelt variety BILBERRY

| Year | Detail of Stage |
|---|---|
| Year 2009. | First cross of the original spelt Schwabenkorn (♀) x var. CHORNOBROVA (♂) possessing the purple seed character. Seeds F1 were produced. |
| Year 2010. | Spelt Schwabenkorn (♀) were pollinated by pollen of the plants F1. Seeds BC1 were produced. |
| Year 2011 | Self-pollination of BC1 plants and isolation of seeds with purple color. |
| Year 2012 | Spelt Schwabenkorn (♀) were pollinated by pollen of the plants BC1. Seeds BC2 were produced. |
| Year 2013-2019 | the six sequential broken backcrosses were performed and elite short stem spelt plants with apparent morphological characters of the spelt Schwabenkorn (♀) possessing of the dark-purple seeds were selected. Each following backcross derived self-pollinated plants were compared with complex morphological characters similar to that of the original spelt Schwabenkorn. |
| Year 2020-2021 | Selection and agronomic performance testing of the homozygote plants with uniform morphological characters predecessors of the variety BILBERRY were evaluated in the field trials. Seeds samples increase. |

(continued)

| Year | Detail of Stage |
| --- | --- |
| Year 2022-2023 | Var. BILBERRY Distinction Uniformity Stability tests performance and seeds increase size needed for seed sales. |

Variety BILBERRY was bred and selected using a modified pedigree selection method for any and all of the following characteristics in the field environment: disease resistance, plant type, plant height, head type, straw strength, maturity, grain yield, test weight, and milling and baking characteristics. BILBERRY has shown no variants other than what would normally be expected due to environment.

**DEPOSIT**

Applicant has made a deposit of at least 625 seeds of spelt variety BILBERRY with the NCIMB 44437 prior to the filing date of this application.

In another aspect, an embodiment of the invention concerns grain received from BILBERRY variety. In one of the embodiments, it has the following characteristics.

Nature 780 g/l, moisture - 14.2%, Protein - 15.34%, raw gluten content - 36.88%, dry gluten content -12.5%, gluten index 31, number of falling - 392 sec, grain color from dark -brown to black -

In another aspect, an embodiment of the invention concerns flour from grain received from BILBERRY variety. In one of the embodiment, it has the following characteristics.

Physico-chemical:

Mass fraction of mineral impurities: there is no crunch when chewing the flour.
Humidity, %, not more than: $\approx$ 15.0. Actual indicator: 13.1
Ash content in terms of dry matter, %, not more: not less than 0.07% lower than the ash content of the grain before cleaning, but not more than 2.0%
Whiteness, conditional units of the RZ-BPL device: not limited

Fineness of grinding, %:

- residue on a wire mesh sieve according to TU 14-4-1374-86, no more than 10, mesh No. 067
- passing through a sieve made of silk fabric in accordance with GOST 4403, not less than 25, fabric No. 38 or No. 41/43 PA

Raw gluten:

- number, %, not less: 18. Actual indicator: 30.85
- quality: not lower than the 2nd group. Actual indicator: 85

Number of drop, s, not less than: 105. Actual indicator: 385
Metallomagnetic admixture, mg in 1 kg of flour, no more than: 3.0

Organoleptic:
Appearance:

Color: white with a grayish tint with noticeable particles of dark brown shells.
Odor: characteristic of wheat flour, without extraneous smell, not musty, not moldy.
Taste: Characteristic of wheat flour, without extraneous aftertaste, not sour, not bitter.

In another aspect the invention concerns food product produced from spelt grain where initial grain colour with violet subtones and higher anthocyanin content conferred from grain, providing attractive and unique external appearance while preserving all useful properties of spelt grain: high protein content more than 15 %), absence of yeast.

In one of the embodiment, food product is spelt bread without adding yeast. The benefit of the proposed decision is that it provides the possibility to use spelt both as flour and sourdough, providing perfect organoleptic results.

The recipe of bread includes:

☐ Wholemeal milled flour from Bilberry black spelt, kg 94,953
☐ Natural honey, kg 5,531
☐ Refined sunflower oil, kg 5,366
☐ Rye flour, refined, kg 5,047
☐ Salt, table salt, food grade, kg 2,308
☐ Drinking water, kg by calculation 93,601 kg
Characteristics of the product: unleavened black spelt bread weighing 0.300 kg with a flour moisture content of 14.5 % is 172.066 %.

In one embodiment, the following process is used for preparing dough:

1. Mixing all elements to bring the dough to the right consistency
2. Resting and stretching & folding or kneading the dough
3. Rise/ fermentation/ proof
4. Pre-heating and preparing the oven
5. Slashing/ scoring the loaf with a sharp knife or razor
6. Baking in the oven
7. Cooling

As a result, the bread with the following characteristics has been obtained:

Appearance (shape): corresponds to the type of product, in the form of an inverted trapezoid of elongated shape, without side spills.
Surface: corresponds to the type of product without cracks and contamination.
Colour: brown with a slight purple tint, without burning.
Sectional view: brown baked semi-finished product with a slight purple tint.
Crumb condition: corresponds to the type of product. Baked, elastic, not wet to the touch, without traces of unleavened dough.
Taste and smell: typical of the product.
No foreign taste or smell is allowed.

Appearance:

Form: Correct, conforming to the form, without breaks.
The surface: corresponds to the type of product, not burnt, without cracks and tears (5).
Color: From light brown to dark brown with a slight purple tint. The color of the lower crust may differ from the color of the upper and side crust (5).
Cross-section: well-baked product, soft brown color with a slight purple tint, without tempering and traces of non-baking (5).
Taste and smell: characteristic of this product, without extraneous taste and smell (5).

As a control sample, the conventional wheat bread has been used.

Organoleptic analysis:

|  | Appearance | Aroma | Taste | Colour | Σ medium |
|---|---|---|---|---|---|
| Control | 5 | 5 | 5 | 5 | 5,0 |
| Experiment 1 1(100%) | 5 | 5 | 5 | 5 | 5,0 |

**Claims**

1. A food product comprising a seed, a plant part or a flour obtained from a seed of a Triticum spelta (Bilberry) variety, wherein representative seed of said variety has been deposited under Number NCIMB 44437.

2. The food product of claim 1, wherein said Tritcum spelta (Bilberry) variety has an anthocyanin content of 60-80 mg/kg; whereby it acts as an antiallergenic.

3. A converted seed, plant, plant part or plant cell product obtained from a Triticum spelta (Bilberry) variety, wherein representative seed of the variety (Bilberry) having been deposited under number NCIMB 44437, wherein the converted seed, plant, plant part or plant cell comprises a locus conversion, and wherein the plant or a plant grown from the converted seed, plant part or plant cell comprises the locus conversion and otherwise comprises essentially all of the physiological and morphological characteristics of the seed, plant, plant part or plant cell of variety (Bilberry) when grown under the same environmental conditions.

4. A method of producing a genetic marker profile comprising extracting nucleic acids from the seed of claim 1 or the plant germinated from said seed and genotyping said nucleic acids at one or more genetic loci, thereby producing a genetic marker profile.

5. A method of plant breeding comprising a) isolating nucleic acids from the seed of claim 1 or a plant germinated from said seed, b) identifying one or more polymorphisms from the isolated nucleic acids, and c) selecting said seed or a plant obtained from said seed having said one or more polymorphisms, wherein the seed or plant is used in a plant breeding method.

6. A method of producing bread by selecting flour derived from a seed of a Triticum spelta (Bilberry) variety, wherein representative seed of said variety has been deposited under Number NCIMB 44437.

7. A seed of Triticum spelta (Bilberry) variety, wherein representative seed of said variety has been deposited under Number NCIMB 44437.

8. A plant or part of the plant of variety (Bilberry), wherein representative seed of said variety (Bilberry) has been deposited under Number NCIMB 44437.

9. A seed produced on the plant of claim 8.

10. A process for producing seed, said process comprising crossing the plant of claim 8 with itself, and harvesting the seed.

11. An F1 seed produced by the process of claim 10 or an F1 plant produced by germinating the seed of claim 7.

12. A method of producing a genetic marker profile comprising extracting nucleic acids from the seed of claim 7 or the plant germinated from said seed and genotyping said nucleic acids at one or more genetic loci, thereby producing a genetic marker profile.

13. A method of plant breeding comprising a) isolating nucleic acids from the seed of claim 7 or a plant germinated from said seed, b) identifying one or more polymorphisms from the isolated nucleic acids, and c) selecting said seed or a plant obtained from said seed having said one or more polymorphisms, wherein the seed or plant is used in a plant breeding method.

14. A plant produced by the process of claim 13.

15. A method of producing a plant derived from variety NCIMB 44437, the method comprising the steps of (a) growing the plant of claim 14; (b) crossing said plant with itself to produce a seed of a progeny plant; (c) repeating step (b) at least one or more times; and (d) growing said progeny plant from said seed and crossing the progeny plant with itself to produce a plant derived from variety NCIMB 44437.

EP 4 725 303 A1

Cyanidin chloride    Delphinidin chloride    Malvidin chloride

Pelargonidin chloride   Peonidin chloride    Petunidine chloride

**Fig.1**

25

Fig.2

Europäisches
Patentamt

European
Patent Office

Office européen
des brevets

## EUROPEAN SEARCH REPORT

**Application Number**

**EP 24 20 6058**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Crop: Spelt Wheat", , 28 March 2024 (2024-03-28), XP093250993, Retrieved from the Internet: URL:https://www.niab.com/sites/default/fil es/imce_uploads/Spelt%20Wheat%20Descriptio ns%202024.pdf * the whole document * ----- | 1-15 | INV. A01H5/10 A01H6/46 |
| X | Olena Biliavets: , 27 October 2023 (2023-10-27), XP93250685, Retrieved from the Internet: URL:https://www.instagram.com/biliavets_ol ena/reel/Cy6knggsmVN/ [retrieved on 2025-02-15] * the whole document * -& Anonymous: "Black-grain Spelt The world's first black-grain spelt", , 1 January 2022 (2022-01-01), XP093251142, Retrieved from the Internet: URL:https://www.biligrainfood.com/products /spelled/ ----- | 1-15 | |
| | | | **TECHNICAL FIELDS SEARCHED (IPC)** A01H C12Q |
| A | MOSKALETS VALENTYN ET AL: "Extension of the forming process in the selection of winter common wheat for productivity and quality by using the gene pool of related wheat species within the framework of food security", SCIENTIFIC HORIZONS, vol. 26, no. 6, 13 June 2023 (2023-06-13), XP093251000, ISSN: 2709-8877, DOI: 10.48077/scihor6.2023.43 ----- | 1-15 | |

-/--

The present search report has been drawn up for all claims

| Place of search | Date of completion of the search | Examiner |
|---|---|---|
| Munich | 17 February 2025 | Marchesini, Patrizia |

EPO FORM 1503 03.82 (P04C01)

Europäisches
Patentamt

European
Patent Office

Office européen
des brevets

**EUROPEAN SEARCH REPORT**

Application Number

EP 24 20 6058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MOSKALETS V. V. ET AL: "Biochemical and molecular-genetic markers of adaptability and quality of genotypes in cultural and wild cereal plants", UKRAINIAN JOURNAL OF ECOLOGY, vol. 9, no. 4, 1 January 2019 (2019-01-01), pages 695-703, XP093251053, ISSN: 2520-2138, DOI: 10.15421/2019_812 Retrieved from the Internet: URL:https://www.ujecology.com/articles/biochemical-and-moleculargenetic-markers-of-adaptability-and-quality-of-genotypes-in-cultural-and-wild-cereal-plants.pdf> ----- | 1-15 | |
| A | AMITEYE SAMUEL: "Basic concepts and methodologies of DNA marker systems in plant molecular breeding", HELIYON, vol. 7, no. 10, 1 October 2021 (2021-10-01), page e08093, XP093250779, GB ISSN: 2405-8440, DOI: 10.1016/j.heliyon.2021.e08093 ----- | 1-15 | TECHNICAL FIELDS SEARCHED (IPC) |

The present search report has been drawn up for all claims

| Place of search | Date of completion of the search | Examiner |
|---|---|---|
| Munich | 17 February 2025 | Marchesini, Patrizia |

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 100334944 C
- DE 19809547 A1
- US 8809654 B
- US 9282712 B
- WO 2009114321 A
- US 20110145940 A
- CA 2977088, Cermak
- WO 2015026883 A1
- US 5107065 A
- US 5453566 A
- US 5759829 A
- WO 03076574 A
- WO 9925853 A
- WO 0152620 A
- WO 03048345 A
- WO 0042219 A
- US 20110296556 A
- US 8809554 B
- WO 5188960 A
- WO 5689052 A
- WO 5880275 A
- WO 8809654 A
- WO 9114778 A
- WO 9931248 A
- WO 0112731 A
- WO 9924581 A
- WO 9740162 A
- US 7605304 B
- US 7696412 B
- US 20040091505 A
- US 5266317 A
- US 5580852 A
- US 5607914 A
- US 20020166141 A
- US 5792931 A
- US PATENTPUBLICATIONSERIALNO A
- WO 03000863 A
- US 20030041348 A
- WO 03033651 A
- US 5605011 A
- US 5013659 A
- US 5141870 A
- US 5767361 A
- US 5731180 A
- US 5304732 A
- US 4761373 A
- US 5331107 A
- US 5928937 A
- US 5378824 A
- WO 9633270 A
- US 4769061 A
- US 4975374 A
- US 4940835 A
- US 5776760 A
- US 5463175 A
- US 5627061 A
- US 6566587 B
- US 6338961 B
- US 6248876 B1
- US 6040497 A
- US 5804425 A
- US 5633435 A
- US 5145783 A
- US 4971908 A
- US 5312910 A
- US 5188642 A
- US 5866775 A
- US 6225114 B1
- US 6130366 A
- US 5310667 A
- US 4535060 A
- US 5633448 A
- US 5510471 A
- US RE36449 E
- US 37287 E
- US 5491288 A
- US 20040082770 A
- EP 1173580 A
- WO 0166704 A
- EP 1173581 A
- EP 1173582 A
- EP 0242246 A
- EP 0242236 A
- US 5969213 A
- US 5489520 A
- US 5550318 A
- US 5874265 A
- US 5919675 A
- US 5561236 A
- US 5648477 A
- US 5646024 A
- US 6177616 B
- US 5879903 A
- US 4810648 A
- US 6288306 B
- US 6282837 B
- US 5767373 A
- WO 0112825 A

- US 6787683 B
- US 6531648 B
- US 6423886 B
- US 6232529 B
- US 6197561 B
- US 6825397 B
- US 20030079247 A
- US 20030204870 A
- US 20040034886 A
- WO 0242424 A
- WO 9822604 A
- WO 03011015 A
- WO 02057439 A
- WO 9910498 A
- WO 0068393 A
- WO 03082899 A
- US 6803498 B
- US 6127600 A
- US 6194638 B
- US 6346403 B
- US 6080913 A
- US 5990389 A
- US 5939599 A
- US 5912414 A
- US 5850016 A
- US 5885802 A
- US 5885801 A
- US 5633436 A
- US 5559223 A
- US 6664445 B
- US 6459019 B
- US 6399859 B
- US 6441274 B
- WO 9940209 A
- WO 9929882 A
- WO 9820133 A
- WO 9601905 A
- WO 9856935 A
- WO 9845458 A
- WO 9842831 A
- WO 9515392 A
- WO 0179516 A
- WO 0009706 A
- US 20030150014 A
- US 20030163838 A
- US 20040068767 A
- US 20040025203 A
- US 4654465 A
- US 4727219 A
- US 3861709 A
- US 3710511 A
- US 5432068 A
- US 5859341 A
- US 6297426 B
- US 5478369 A
- US 5824524 A
- US 5850014 A
- US 6265640 B
- US 5892009 A
- US 5965705 A
- US 5929305 A
- US 5891859 A
- US 6417428 B
- US 6664446 B
- US 6706866 B
- US 6717034 B
- US 6801104 B
- US 6177275 B
- US 6107547 A
- US 6084153 A
- US 20040148654 A
- US 20040237147 A
- US 20030166197 A
- US 29CA2977088 A
- US 2017082320040128719 A
- US 20040098764 A
- US 20040078852 A
- WO 2000060089 A
- WO 2001026459 A
- WO 2001035725 A
- WO 0073475 A
- WO 2001034726 A
- WO 2001035727 A
- WO 2001036444 A
- WO 2001036597 A
- WO 2001036598 A
- WO 2002015675 A
- WO 2002017430 A
- WO 2002077185 A
- WO 2002079403 A
- WO 2003013227 A
- WO 2003013228 A
- WO 2003014327 A
- WO 2004031349 A
- WO 2004076638 A
- WO 9809521 A
- WO 0136596 A
- WO 9938977 A
- WO 2000006341 A
- WO 04090143 A
- WO 0202776 A
- WO 2003052063 A
- WO 0164898 A
- WO 200032761 A
- WO 9749811 A, LHY
- WO 9856918 A
- WO 9710339 A
- US 6573430 B
- US 6713663 B
- WO 9614414 A
- WO 9638560 A
- WO 0121822 A
- WO 0044918 A
- WO 9949064 A
- WO 0046358 A
- WO 9729123 A

- US 6794560 B
- US 6307126 B
- WO 9909174 A
- US 5981842 A
- US 5780709 A
- WO 9219731 A


**Non-patent literature cited in the description**

- **ABDEL-AAL E.-S. M** ; **HUCL**. A Rapid Method for quantifying Total Anthocyanins in Blue Aleurone and Purple Pericarp Wheats. *Cereal Chem.*, 1999, vol. 76 (3), 350-354
- **GAO et al.** *Plant Journal*, 2010, vol. 1, 176-187
- **URNOV et al.** *Nat Rev Genet.*, 2010, vol. 11 (9), 636-46
- **SHUKLA et al.** *Nature*, 2009, vol. 459 (7245), 437-41
- *Nucleic Acids Res.*, 2011, vol. 39 (12)
- **BOCH et al.** *Science*, 2009, vol. 326 (5959), 1509-12
- **BELHAJ et al.** *Plant Methods*, 2013, vol. 9, 39
- **HARDENBOL et al.** *Nat Biotech*, 2003, vol. 21, 673-678
- **METZKER**. *Nat*, 2010, vol. 24
- *Rev Genet*, vol. 11, 31-46
- **EGAN et al.** *Am J Bot*, 2012, vol. 99, 175-185
- **ELSHIRE et al.** *PLoS ONE*, 2011, vol. 6, e19379
- Hybrid Wheat. **K.A. LUCKEN**. In Wheat and Wheat Improvement. 1987, 444-452
- The Pesticide Manual: A World Compendium. British Crop Production Council.
- **ABDEL-AAL E.-S. M.** ; **HUCL P. A.** Rapid Method for quantifying Total Anthocyanins in Blue Aleurone and Purple Pericarp Wheats. *Cereal Chem.*, 1999, vol. 76 (3), 350-354